(19) Europäisches Patentamt
European Patent Office
Office européen des brevets

(11) **EP 2 674 777 A1**

(12) **EUROPEAN PATENT APPLICATION**

(43) Date of publication:
**18.12.2013 Bulletin 2013/51**

(51) Int Cl.:
*G01S 7/52* (2006.01)   *G01S 15/89* (2006.01)

(21) Application number: **13171576.5**

(22) Date of filing: **12.06.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **13.06.2012 KR 20120063401**

(71) Applicant: **Samsung Electronics Co., Ltd Gyeonggi-do 443-742 (KR)**

(72) Inventors:
• **Kim, Bae-hyung**
  **446-712 Gyeonggi-do (KR)**

• **Kim, Dong-wook**
  **446-712 Gyeonggi-do (KR)**
• **Song, Jong-keun**
  **446-712 Gyeonggi-do (KR)**
• **Lee, Seung-heun**
  **446-712 Gyeonggi-do (KR)**
• **Cho, Kyung-il**
  **446-712 Gyeonggi-do (KR)**

(74) Representative: **Grünecker, Kinkeldey, Stockmair & Schwanhäusser Leopoldstrasse 4 80802 München (DE)**

(54) **Method and apparatus for performing 3-dimensional ultrasound volume scanning by using 2-dimensional transducer array**

(57)     A method and apparatus for performing three-dimensional (3D) ultrasound volume scanning by using a two-dimensional (2D) transducer array, in which a plurality of transducer elements are two-dimensionally arranged, including applying at least two codes which are orthogonal to each other to at least one one-dimensional (1D) transducer array which is included in the 2D transducer array, the 1D transducer array having transducer elements which are arranged linearly from among the plurality of the transducer element; obtaining signals which respectively correspond to the codes which are orthogonal to each other from signals which are reflected by a target object and received by the plurality of transducer elements; and generating image data regarding the target object by using the obtained signals.

FIG. 6

**Description**

BACKGROUND

**1. Field**

Exemplary embodiments relate to an ultrasound imaging method and an apparatus therefor, and more particularly, to a method and an apparatus for performing three-dimensional ultrasound volume scanning by using a two-dimensional transducer array.

**2. Description of the Related Art**

In an ultrasound diagnostic device, a probe has a plurality of transducers. When ultrasound waves which have frequencies ranging from several Hz to hundreds of MHz are transmitted from a probe of a three-dimensional (3D) imaging device to a particular region in the body of a patient, the ultrasound waves are partially reflected by various different tissues. In particular, ultrasound waves are reflected differently based on changes in densities of regions inside the body, e.g., blood cells in blood plasma, small structures inside organs, and/or other factors. The reflected ultrasound waves cause oscillations in the transducer of the probe, and the transducer outputs electrical pulses based on the oscillations. The electrical pulses are converted to form an image.

A recently developed ultrasound scanner forms an ultrasound beam by focusing ultrasound waves by using between 64 and 256 transducers. The ultrasound beam is steered electrically rather than mechanically. In a one-dimensional (1D) transducer array, a plurality of transducers are linearly arranged, and therefore, the ultrasound beam is steerable only in a lateral direction. As a result, the ultrasound beam cannot be steered in an elevation direction. Thus, only two-dimensional (2D) images may be obtained.

In a 2D transducer array in which transducers are arranged both in an elevation direction and in a lateral direction, an ultrasound beam may be steered both in the elevation direction and in the lateral direction, and thus dynamic focusing may be performed both in the elevation direction and in the lateral direction. As a result, devices which are capable of obtaining 3D volume ultrasound images have been introduced.

SUMMARY

Provided are methods and apparatuses for obtaining three-dimensional (3D) volume ultrasound images in real time by using a relatively small number of transducers and reducing an amount of data processed per hour.

Provided are computer readable recording media having recorded thereon computer programs for executing the methods on a computer.

Additional aspects will be set forth in part in the description which follows and, in part, will be apparent from the description, or may be learned by practice of the presented exemplary embodiments.

According to an aspect of one or more exemplary embodiments, a method for performing three-dimensional (3D) ultrasound volume scanning by using a two-dimensional (2D) transducer array, in which a plurality of transducer elements are two-dimensionally arranged, the method includes applying at least two codes that are orthogonal to each other to at least one one-dimensional (1D) transducer array which is included in the 2D transducer array, the at least one 1D transducer array having at least two transducer elements which are arranged linearly from among the plurality of transducer elements; obtaining signals which respectively correspond to the applied at least two codes that are orthogonal to each other from signals which are reflected by a target object and received by the plurality of transducer elements; and generating image data which relates to the target object by using the obtained signals. In particular, being orthogonal indicates that auto-correlation is equal to one and cross-correlation is equal to zero.

According to another aspect of one or more exemplary embodiments, there is provided a non-transitory computer readable recording medium having recorded thereon a computer program for implementing the above method.

According to another aspect of one or more exemplary embodiments, a three-dimensional (3D) ultrasound volume scanning apparatus includes a two-dimensional (2D) transducer array, in which a plurality of transducer elements are two-dimensionally arranged; a transmitter which is configured to apply at least two code that are orthogonal to each other to at least one one-dimensional (1D) transducer array, the at least one 1D transducer array having at least two transducer elements which are arranged linearly from among the plurality of transducer elements; a receiver which is configured to obtain signals which respectively correspond to each other from signals which are reflected by a target object and received by the plurality of transducer elements; and an image processor which is configured to generate image data which relates to the target object by using the obtained signals.

BRIEF DESCRIPTION OF THE DRAWINGS

These and/or other aspects will become apparent and more readily appreciated from the following description of exemplary embodiments, taken in conjunction with the accompanying drawings of which:

FIG. 1 is a diagram which illustrates an ultrasound imaging system, according to an exemplary embodiment;

FIG. 2 is a diagram which illustrates a 1D transducer array which is configured to obtain image data with respect to one pixel of a 2D image;

FIG. 3 is a diagram which illustrates a transmission beam plane or a reception beam plane which is formed of a 1D transducer array in an elevation direction;

FIG. 4 is a diagram which illustrates dynamic beam focusing in a 1D transducer array;

FIG. 5 is a diagram which illustrates formation of a scan line by using cross transducer arrays;

FIG. 6 is a block diagram which illustrates the probe and the ultrasound volume scanning device shown in FIG. 1;

FIGS. 7A, 7B, 7C, and 7D are diagrams which illustrate 2D transducer arrays, according to exemplary embodiments;

FIG. 8 is a diagram which illustrates a 1D transducer array which is included in a 2D transducer array according to an exemplary embodiment which is configured to form a beam plane;

FIG. 9 is a diagram which illustrates the structure of a reception decoder;

FIG. 10 is a flowchart which illustrates a method for scanning a 3D ultrasound volume by using a cross-transducer array;

FIG. 11 is a diagram which illustrates dynamic focusing of an ultrasound beam using a plurality of 1D transducer arrays, according to an exemplary embodiment;

FIG. 12 is a diagram which illustrates structures of the transmitter and the receiver, according to an exemplary embodiment;

FIG. 13A is a diagram which illustrates an example of type A Golay codes;

FIG. 13B is a diagram which illustrates an example of type B Golay codes;

FIG. 13C is a diagram which illustrates sound waves which are generated by a transducer when the code shown in FIG. 13A is output by the pulser 601.

FIG. 13D is a diagram which illustrates sound waves which are generated by a transducer when the code shown in FIG. 13B is output by the pulser 601.

FIG. 14 is a diagram which illustrates a 2D transducer array which is configured to transmit two types of code to a focus point;

FIG. 15 is a diagram which illustrates the cross-transducer arrays of FIG. 14 as being configured to receive two types of code;

FIG. 16 is a diagram which illustrates the structure of a reception decoder;

FIG. 17 is a flowchart which illustrates a method for scanning a 3D ultrasound volume by using a cross-transducer array; and

FIG. 18 is a flowchart which illustrates a method for scanning a 3D ultrasound volume by using the cross-transducer array shown in FIG. 14.

DETAILED DESCRIPTION

Reference will now be made in detail to exemplary embodiments, examples of which are illustrated in the accompanying drawings, wherein like reference numerals refer to like elements throughout. In this regard, the present exemplary embodiments may have different forms and should not be construed as being limited to the descriptions set forth herein. Accordingly, the exemplary embodiments are merely described below, by referring to the figures, to explain aspects of the present disclosure.

Hereinafter, with reference to the accompanying drawings, an exemplary embodiment will be described in detail.

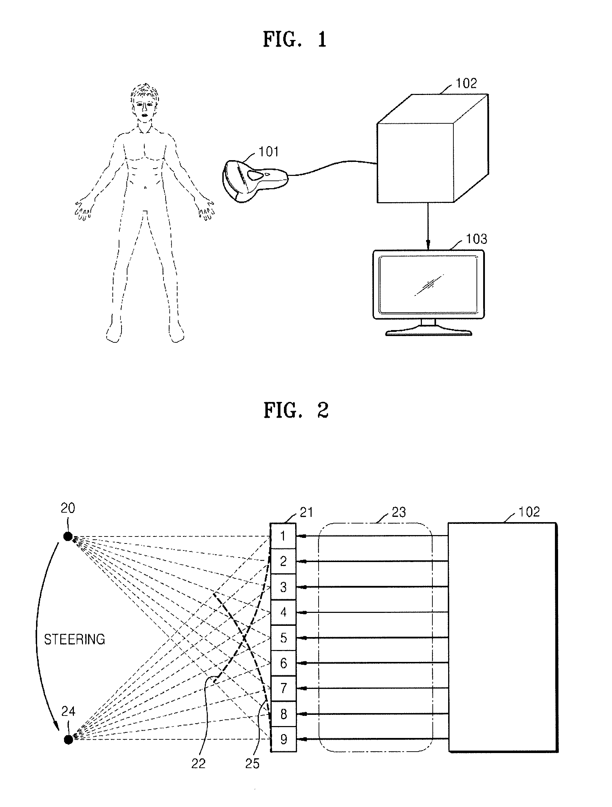

FIG. 1 is a diagram which illustrates an ultrasound imaging system, according to an exemplary embodiment. Referring to FIG. 1, the ultrasound imaging system shown in FIG. 1 includes a probe 101, an ultrasound volume scanning device 102, and an image display device 103. The probe 101 includes a two-dimensional (2D) transducer array. The 2D transducer array comprises two 1D transducer arrays which are respectively arranged in an elevation direction and in a lateral direction. The two 1D transducer arrays intersect each other. When a corresponding electrical signal is input to each of the transducers included in the 1D transducer arrays of the probe 101 by the ultrasound volume scanning device 102, each of the transducers converts the electrical signal to a source signal for detecting image data which relates to the interior of a target object.

In particular, the target object may be a human body, an animal body, a metal object, or any other suitable target object. Hereinafter, exemplary embodiments will be described assuming that the target object is a human body. However, it will be understood by one of ordinary skill in the art that the target object in the exemplary embodiment shown in FIG. 1 and the exemplary embodiments to be described below may also be an animal body or a metal object, for example.

Further, the probe 101 uses a plurality of transducer array elements which are similar to those of the transducer array instead of using just one transducer, because an intensity of a source signal which is generated by one transducer is not strong enough to detect image data which relates to the inside of a target object. Therefore, both of an intensity of a source signal and a resolution of an image may be improved by condensing signals to a desired point by using a plurality of transducers.

As shown in FIG. 1, the ultrasound volume scanning device 102 may be embodied as a separate device from the probe 101. It will be understood by one of ordinary skill in the art that the probe 101 and the ultrasound volume scanning device 102 may be embodied as a single device. The ultrasound volume scanning device 102 may generate a 2D medical image as described below. Source signals which are received from the probe 101 are focused towards a first focal point inside a human body, and the ultrasound volume scanning device 102 detects image data of a pixel which corresponds to the first focal point from a reflected signal that is formed due to reflection of the focused source signals. Next, source signals which are received from the probe 101 are focused towards a second focal point inside the human body, and the ultrasound volume scanning device 102 detects image data of a pixel which corresponds to the second focal point from a reflected signal that is formed as the focused source signals are reflected. The ultrasound volume scanning device 102 detects image data of pixels constituting a 2D imaging region representative of the inside the human body by repeating the above-described processes with respect to all points constituting the 2D imaging region representative of the inside the human body, and may generate a medical image of the 2D imaging region representative of the inside the human body by merging the detected image data of the pixels.

FIG. 2 is a diagram which illustrates a 1D transducer array 21 which is configured to obtain image data with respect to one pixel of a 2D image. In FIG. 2, the 1D transducer array 21 includes nine transducers that are linearly arranged, and the transducers are numbered in an order starting from 1 above to 9 below. However, the definition of the term "linear" is not limited to a straight line herein, and may include a curved line. For example, a modification may be made to the present exemplary embodiment to form a closed-curve, that is, an annular array by increasing curvature of a 1D transducer array, according to an exemplary embodiment. A focal point 20 refers to a point at which source signals are focused. Because image data is obtained from the focal point, the focal point corresponds to a pixel of the 2D image. The ultrasound volume scanning device 102 outputs a plurality of electrical signals 23 that may be converted to source signals by transducers of the transducer array 21 inside the probe 101. The number of electrical signals output by the ultrasound volume scanning device 102 is the same as the number of transducers inside the probe 101. In FIG. 2, the ultrasound volume scanning device 102 outputs nine electrical signals to which there are nine transmitting transducers arranged. In particular, in order to focus the nine source signals generated by the probe 101 at a focal point 20 at the same time, it is necessary for the source signals to arrive at the focal point 20 simultaneously. Meanwhile, distances between the arbitrary focal point 20 and respective transducers are different from one another. Therefore, if it is assumed that transducers generate source signals simultaneously, the source signals arrive at the arbitrary focal point 20 at different points of time, due to the different distances between the arbitrary focal point 20 and the respective transducers. In other words, as shown in FIG. 2, a first transducer of the transducer array 21 is the closest to the focal point 20, and respective distances between the focal point 20 and the remaining transducers increase in the order from the second transducer to the ninth transducer. Accordingly, for a plurality of source signals generated by the probe 101 to arrive at the focal point 20 simultaneously, it is necessary for a transducer which is relatively far from the focal point 20 to generate a source signal before a transducer which is relatively close to the focal point 20 generates a source signal. In this regard, it is necessary for the first transducer to generate a source signal last. Lines connecting positions of signals generated by other transducers when the first transducer generates a source signal constitute a delay pattern 22 which corresponds to the focal point 20.

In particular, in order for transducers inside the probe 101 to generate source signals at different points of time, it is necessary to input the plurality of electrical signals 23 (in FIG. 2, nine electrical signals) to respective transducers inside the probe 101 at different points of time.

As described above, the probe 101 outputs the nine electrical signals as nine source signals. In particular, the ultrasound volume scanning device 102 delays electrical signals to be input to the transducers by periods of time which are inversely proportional to differences in distances between the focal point 20 and the transducers, respectively, and outputs the electrical signals, and thus source signals output by the transducers may be focused at the one focal point 20. An electrical signal is delayed by a particular period of time and input to a corresponding transducer. An electrical signal which is input to the transducer 9 which is farthest from the focal point 20 may be input without being delayed. Controlling the probe 101 so that a plurality of source signals arrive at the arbitrary focal point 20 simultaneously is referred to as transmission beamforming.

The beamforming may not only be applied to transmission, but may also be applied to reception of signals by the ultrasound volume scanning device 102. Source signals transmitted by the transducer array 21 are merged at the focal point 20 via the transmission beamforming as described above, and the merged source signals are reflected by the focal point 20 and returned to the transducer array 21. Each of the transducers of the transducer array 21 converts a reflected signal back to an electrical signal and outputs the electrical signal to the ultrasound volume scanning device 102. As described above, because intensities of the converted electrical signals are very weak, the ultrasound volume scanning device 102 does not use each of the electrical signals individually to form an image, but instead forms an image by merging the electrical signals into one signal. However, due to differences in respective distances between the transducers and the focal point 20, the reflected source signals respectively arrive at the corresponding transducers at different points of time, and thus the transducers generate electrical signals at different points of time, respectively.

Therefore, in order to merge electrical signals output by the transducers into one signal, the ultrasound volume scanning device 102 delays the electrical signals output by the transducers respectively by periods of time which are inversely proportional to respective differences in distances between the transducers and the focal point 20, and merges the electrical signals output by the transducers when electrical signals are output by all of the transducers. In particular, because an electrical signal which is input to the transducer 9 which is farthest from the focal point 20 is input last from among electrical signals input to the transducers of the transducer array 21, the particular electrical signal may be merged with electrical signals output by the other transducers without being delayed.

Merging the electrical signals from the transducers of the transducer array 21 in consideration of time differences between the electrical signals as described above is referred to as reception beamforming.

The ultrasound volume scanning device 102 performs reception beamforming with respect to a plurality of electrical signals output by the transducer array 21, and obtains information regarding brightness at the focal point 20 by using an intensity of a beam-formed signal. Next, the ultrasound volume scanning device 102 generates a 2D medical image by repeatedly performing the processes described above with respect to a plurality of points in a 2D imaging region inside the human body. Such a 2D medical image which is generated based on the brightness information is referred to as a B mode image. However, it would have been obvious to one of ordinary skill in the art to use similar processes for generating not only a B mode image, but also an A mode image and a M mode image, and thus detailed descriptions thereof will be omitted. The generated medical image is transmitted to the image display device 103. Although a 2D image may be generated by using the 1D transducer array 21 of FIG. 2, a 3D ultrasound volume image may be obtained by using a cross-transducer array, according to an exemplary embodiment.

The image display device 103 receives the medical image from the ultrasound volume scanning device 102 and displays the medical image.

Further, the focal point 20 of the transmission or reception beamforming may be switched to another focal point 24 via phase shift or time delay of a transmitted signal or a received signal. The switching is referred to as steering of an ultrasound beam. Hereinafter, the term "delay" will be interpreted to include both phase shift and time delay. In case of steering of transmission beamforming, delay patterns applied to the transducer array 21 vary based on locations of focal points, and thus multiple focal points may not be formed at the same time in a general transmission beamforming. For example, after transmission beamforming is completed with respect to the focal point 20, transmission beamforming is performed with respect to another focal point 24. During steering of transmission beamforming, a number of times for forming transmission beams increases as a number of focal points increases, and thus a period of time elapsed for obtaining a single 3D image increases in proportion to the number of times for forming transmission beams. Conversely, in a case of steering of reception beamforming, signals received by transducers of the transducer array 21 may be stored in a recording medium, such as a memory, and the delay pattern 22 and the delay pattern 25 may be applied to the stored signals in parallel. Therefore, a period of time which is required for obtaining a 3D image does not increase. In particular, steering of reception beamforming may be considered as changing delay patterns while received signals are being processed.

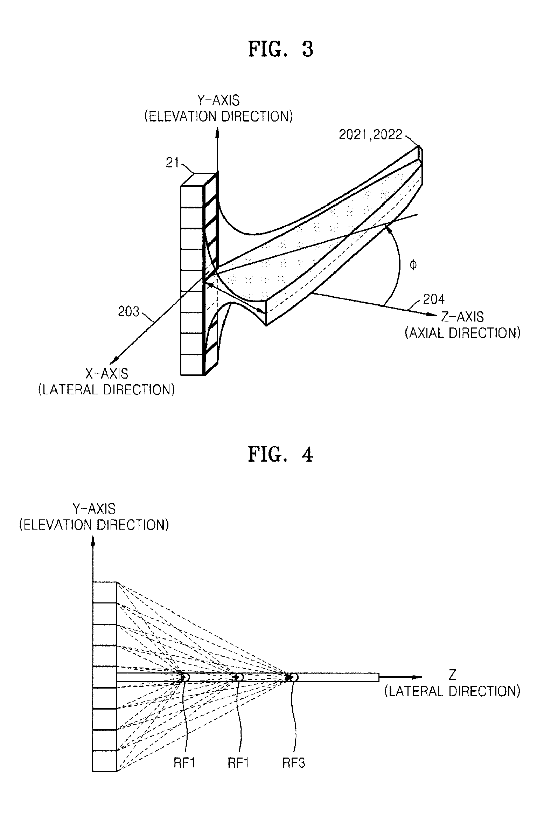

FIG. 3 is a diagram which illustrates a transmission beam plane 2021 or a reception beam plane 2022 which is formed by a 1D transducer array 21 in an elevation direction. In FIG. 3, each of the cubes indicates a transducer, whereas a plurality of transducers combined in series indicate the transducer array 21. When an electrical signal is applied to each of the transducers, according to Huygens' principle, a source signal is propagated from the transducer in an eventail shape (i.e., a fan shape). When the beamforming described above is performed as shown in FIG. 2, source signals propagated from the transducers in eventail shape are merged. Referring to FIG. 3, the merged source signals form an eventail shape plane having a radius r from the transducer at the center of the transducer array 21. The plane is referred to as the transmission beam plane 2021. In the related art, U.S. Patent No. 5,305,756 and U.S. Patent No. 5,417,219 disclose focusing a beam on a transmission beam plane or a reception beam plane.

An angle formed between a plane extending in a lateral direction 203 and a depth-wise direction 204 and the transmission beam plane 2021 may be indicated as $\Phi$. Because the depth-wise direction is a direction identical to the axis of an ultrasound beam, the depth-wise direction is considered as being identical to the axial direction. The ultrasound volume scanning device 102 may change the angle $\Phi$ for the transmission beam plane 2021 via steering of the beamforming, as described above with reference to FIG. 2. In the same regard, the reception beam plane 2022 refers to an eventail-shaped plane which is formed while reflected source signals are being beam-formed. The value $\Phi$ for the reception beam plane 2022 may also be changed via the steering. In case of employing transducer arrays that perpendicularly intersect each other, two 1D transducer arrays are arranged to be perpendicular to each other. Because formations of a transmission beam plane and a reception beam plane of the 1D transducer array in a lateral direction are the same as formations of a transmission beam plane and a reception beam plane of the 1D transducer array in an elevation direction, detailed descriptions thereof will be omitted.

FIG. 4 is a diagram which illustrates dynamic beam focusing in a 1D transducer array. The dynamic beam focusing refers to the transducer array focusing an ultrasound beam by changing a focal distance Rf of the ultrasound beam along the axial direction (Z direction) of the beam. If a plurality of transducers form an ultrasound beam to a fixed focal point, resolution in the elevation direction is excellent. However, as a distance gets farther from the focal point, resolution in the elevation direction is deteriorated. Despite that fact, a general ultrasound volume scanning method employs 1-way dynamic focusing for fixedly focusing transmission beams and for dynamically focusing reception beams. Compared to the one-way dynamic focusing, two-way dynamic focusing for dynamically focusing both transmission beams and reception beams may provide superior resolution in the elevation direction. However, in the case of two-way dynamic focusing, ultrasound signals are transmitted for a number of times for obtaining a single 3D image, and a period of time which is required for obtaining the 3D image increases in proportion to the number of times the ultrasound signals are transmitted. Generally, two-way dimensional focusing is not used for obtaining a 3D image in real time. In detail, in a case in which dynamic focusing is performed during transmission, a general ultrasound volume scanning device sequentially performs beamforming by applying delay patterns which respectively correspond to focal distances RF1, RF2, and RF3 to ultrasound signals. Therefore, in dynamic focusing during transmission, a number of times for forming ultrasound beams increases based on a number of focal points. Conversely, in a case in which dynamic focusing is performed during reception, delay patterns which respectively correspond to focal points may be applied to signals in parallel, which are received by a transducer array. Therefore, a period of time which is required for obtaining a 3D image does not increase in dynamic focusing during reception.

Both the steering of the beamforming described above with reference to FIG. 3 and the dynamic focusing of the beamforming described above with reference to FIG. 4 use time delays for focusing ultrasound signals. The difference therebetween is that in FIG. 3, the steering of the beam changes by the angle $\Phi$, and in FIG 4, the dynamic focusing of beam changes the focal distance Pf. However, both of the steering and the dynamic focusing are performed by varying time delays for switching focal points in a space. Different scan lines are formed by the steering, whereas ultrasound beams are focused to different locations on a single scan line by the dynamic focusing.

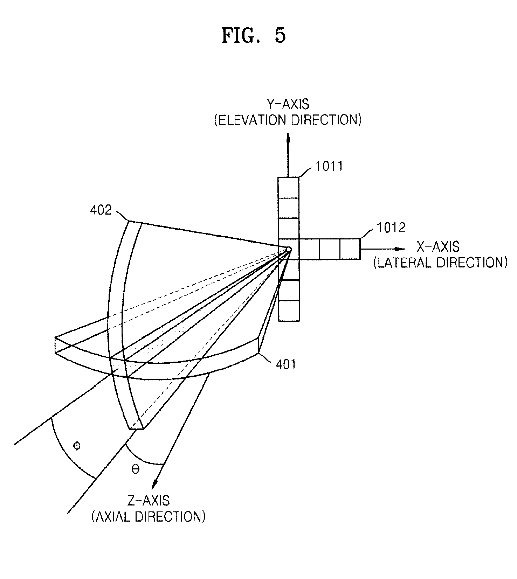

FIG. 5 is a diagram which illustrates formation of a scan line by using cross transducer arrays 1011 and 1012. An example of an apparatus which is configured to implement methods for obtaining a 3D image by using the cross-transducer arrays 1011 and 1012 is a cross-array which uses fixed focusing (CA-FF) in which only one of two transducer arrays transmits signals and the other transducer array only receives signals. A technical configuration for such a cross-array is disclosed in U.S. Patent No. 5,901,708 ("Method and apparatus for forming ultrasonic three-dimensional image using cross array"). In particular, when transmission signal patterns from a pulser are input to transducers of the transducer array 1011 in an elevation direction, source signals to be propagated to a target object are generated. The transducer array 1012 in the lateral direction may receive reflected signals, which are the reflections of the source signals as reflected by the target object, convert the reflected signals to electrical signals, and output the converted electrical signals. Therefore, if the transducer array 1011 in the elevated direction is a transmission array, the transmission beam plane 401 of FIG. 4 is formed. If the transducer array 1012 in the lateral direction is a reception array, the reception beam plane 402 is formed. Hereinafter, it will be assumed that the elevation direction corresponds to the y-axis direction, the lateral direction corresponds to the x-axis direction, and the depth-wise direction corresponds to the z-axis direction. $\Phi$ denotes an angle between an x-z plane and the transmission beam plane 401, whereas $\Theta$ denotes an angle between a y-z plane and the reception beam plane 402. The transmission beam plane 401 which has the angle $\Phi$ may be formed by applying transmission signal patterns which correspond to $\Phi$ to transducers in the transmission array 1011. A straight line at which the transmission beam plane 401 and the reception beam plane 402 intersect each other is formed, where the straight line is a scan line. A scan line refers to a path via which image data may be obtained. In particular, a scan line may be defined by specifying $\Phi$ and $\Theta$. Image data corresponds to a location on a predetermined scan line, and the location corresponds to a focal distance. Therefore, a 3D volume ultrasound image is formed by changing the angles $\Phi$ and $\Theta$ by a predetermined amount (e.g., by 1 degree) by adjusting delays of electrical signals applied to the cross-transducer arrays 1011 and 1012, thereby changing the focal distance.

In particular, a plurality of steered reception beam planes may be simultaneously formed by applying a plurality of reception delay patterns to signals received by the reception array 1012 in parallel.

Further, for the cross-transducer arrays 1011 and 1012 to form a scan line, it is necessary for the transmission beam plane 401 and the reception beam plane 402 to not be formed in parallel. When a plurality of beam planes are formed by adjusting $\Theta$, a plurality of scan lines may be formed with respect to a single transmission beam plane.

Because a 3D volume ultrasound image is obtained via one-way dynamic focusing for fixedly focusing transmission beams and for dynamically focusing reception beams based on the CA-FF technique described above, resolution of the 3D volume ultrasound image in the elevation direction is poor outside the focal distance of the transmission beam.

In this aspect, it is impossible to perform two-way dynamic focusing by using the CA-FF technique. Furthermore, for a 3D volume ultrasound image obtained by using the CA-FF technique, it is necessary to transmit ultrasound beams N times in order to form N transmission beam planes with different Φ values, and thus a period of time which is required for forming a 3D volume ultrasound image via a plurality of beam transmissions increases.

However, even if two-way dynamic focusing and steering of a transmission beam plane are performed, the ultrasound volume scanning device 102 according to the present exemplary embodiment uses codes that are orthogonal to each other, and thus a period of time which is required for obtaining a 3D volume ultrasound image does not increase. Hereinafter, a method of obtaining a 3D volume ultrasound image by applying codes that are orthogonal to each other to a 2D transducer array in correspondence with a plurality of scan lines, a plurality of beam planes, and/or a plurality of focal distances will be described.

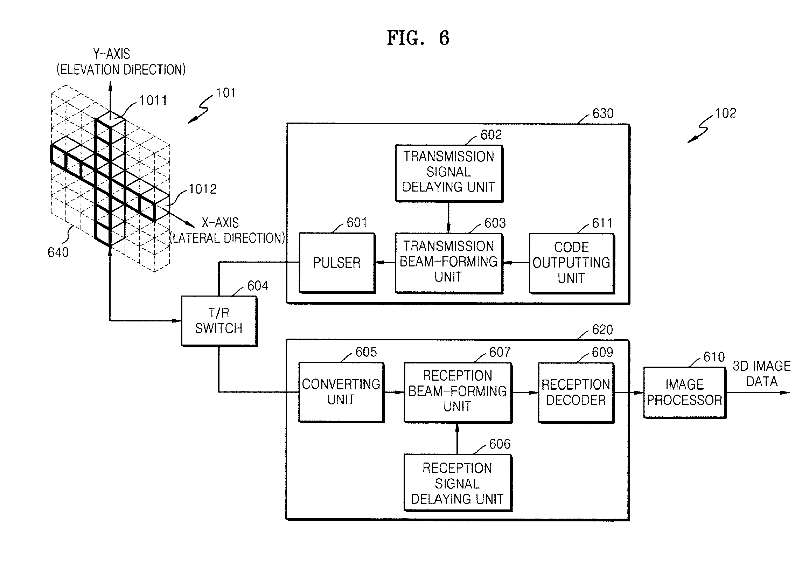

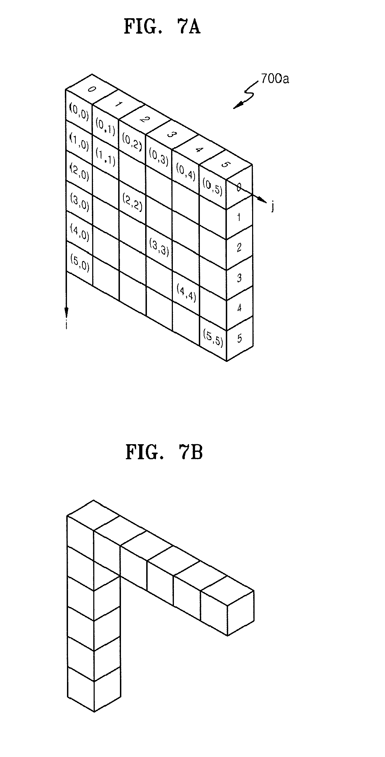

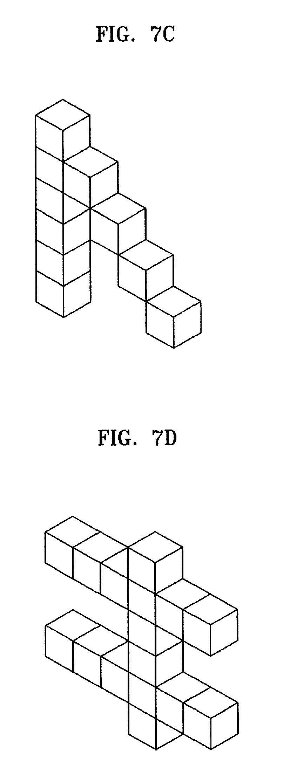

FIG. 6 is a block diagram which illustrates the probe 101 and the ultrasound volume scanning device 102 shown in FIG. 1. A probe 101 according to the present exemplary embodiment includes a 2D transducer array 640 in which a plurality of transducers are two-dimensionally arranged. In the 2D transducer array 640, at least two 1D transducer arrays are arranged in different directions. The 2D transducer array 640 may not only be the cross-transducer array as shown in FIG. 5, but also be in any of various forms as described below with reference to FIGS. 7A, 7B, 7C, and 7D.

The system which comprises the probe 101 and the ultrasound volume scanning device 102, as shown in FIG. 6, includes a 2D transducer array 640, a transmit/receive (T/R) switch 604, a transmitter 630, a receiver 620, and an image processor 610. The transmitter 630 includes a pulser 601, a transmission signal delaying unit 602, a transmission beam-forming unit 603, and a code outputting unit 611, whereas the receiver 620 includes a converting unit 605, a reception signal delaying unit 606, a reception beam-forming unit 607, and a reception decoder 609. Each of the pulser 601, the transmission signal delaying unit 602, the transmission beam-forming unit 603, the code outputting unit 611, the converting unit 605, the reception signal delaying unit 606, the reception beam-forming unit 607, and the reception decoder 609 may be embodied, for example, as a hardware component which includes a microprocessor or integrated circuitry or any other suitable type of hardware, or as a software module which includes instructions which can be executed by a computer in order to perform the corresponding function.

The 2D transducer array 640 is arranged inside the probe 101 and, as shown in FIGS. 5 and 7A, 7B, 7C, and 7D, includes at least two 1D transducer arrays. If the transducer array 640 is a cross-transducer array as shown in FIG. 5, the transducer array 610 includes a transducer array in an elevation direction 1011 and a transducer array in a lateral direction 1012. The cross-transducer arrays 1011 and 1012 intersect each other and may share one transducer at the origin of the x-axis and the y-axis. When transmission signal patterns are received by respective transducers of the 2D transducer array 640 from the pulser 601 via the T/R switch 604, the 2D transducer array 640 converts the transmission signal patterns to source signals. When the signals are reflected by a target object and towards the 2D transducer, the 2D transducer array 640 converts the reflected source signals back to electrical signals.

Further, examples of methods for forming a 3D volume image by using a single 1D transducer array include a free-hand scanning method or a wobbling method for performing mechanical scanning by using a motor. However, resolutions or frame rates of images formed by using the free-hand scanning method or the wobbling method are limited. Therefore, a 2D transducer array may be used to provide a high-resolution 3D image at a high speed. For example, if 96 transducers are arranged in each of an elevation direction and in a lateral direction, a total number of necessary transducers is 96 times 96, that is, 9216. In order to be able to use a general 2D transducer array which includes such a large number of transducers, it is necessary to increase the size of an ultrasound volume scanning device for controlling and analyzing signals which are applied to the respective transducers. As a result, costs for manufacturing the transducer array and the ultrasound volume scanning device increase. According to the present exemplary embodiment, if the cross-transducer arrays 1011 and 1012 include 96 transducers each, a total number of necessary transducers is only 96 times 2, that is, 192. Therefore, an ultrasound volume scanning device and a probe employing the cross-transducer arrays 1011 and 1012 may embodied with a significantly small number of devices as compared to those employing a general 2D transducer array, and thus images of the same quality may be generated at a much lower cost. Furthermore, as a number of arrays increases, a number of cables which are required for interconnecting the probe 101 and the ultrasound volume scanning device 102 also increases, thus increasing the overall cable weight. Generally, an ultrasound diagnosis requires from 20 to 30 minutes on average to complete, and may take longer. Therefore, it is necessary to reduce the overall cable weight.

The pulser 601 may be a bipolar pulser. The pulser 601 receives delayed transmission signal patterns from the transmission beam-forming unit 603, amplifies the transmission signal patterns into bipolar pulses with predetermined voltages, and applies the bipolar pulses to the 2D transducer array 640 via the T/R switch 604. In response to voltages of the bipolar pulses input by the pulser 601, the 2D transducer array 640 generates ultrasound pulses and transmits the ultrasound pulses to a particular location within the human body.

The transmission signal delaying unit 602 stores patterns for delaying ultrasound pulses in order to compensate for differences in points of time at which ultrasound waves arrive at a target object based on corresponding entries which are provided a look-up table in the transmission beam-forming unit 603, where the patterns vary based on positions of transducers of the 2D transducer array 640. The reason for this is that it is necessary to set different points of time at which transmission signal patterns are applied by the pulser 601 to respective transducers in order to change the steering angles and the radius r of an arbitrary transmission beam plane of each of the predetermined 1D transducer arrays which are included in the 2D transducer array 640 (i.e., the beamforming as described above with reference to FIG. 2). Therefore, the transmission signal delaying unit 602 stores tables which are inclusive of points of time at which signal patterns are applied with respect to the steering angles and the focal distance of arbitrary transmission beam planes of each of the predetermined 1D transducer arrays included in the 2D transducer array 640. When the steering angles and the focal distance of transmission beam plane are input by a control unit (not shown), the transmission signal delaying unit 602 outputs a delay look-up table which corresponds to the steering angles to the transmission beam-forming unit 603.

The transmission beam-forming unit 603 receives delay values for arbitrary focal points from the transmission signal delaying unit 602 in the form of a look-up table.

The code outputting unit 611 stores transmission signal patterns to be provided to the transmission beam-forming unit 603. For example, it is assumed below that the code outputting unit 611 stores type A codes and type B codes that are orthogonal to each other and outputs the codes to the transmission beam-forming unit 603.

The T/R switch 604 connects and disconnects the pulser 601, the converting unit 605, and the 2D transducer array 640 to and from one another. When the pulser 601 outputs transmission signal patterns to the 2D transducer array 640, the T/R switch 604 disconnects the converting unit 605. When the 2D transducer array 640 receives reflected signals, generates electrical signals therefrom, and outputs the electrical signals to the converting unit 605, the T/R switch 604 disconnects the pulser 601 and only connects the 2D transducer array 640 and the converting unit 605. In particular, the T/R switch 604 functions as a duplexer for preventing the converting unit 605 from being affected by high voltage power emitted by the pulser 601, and connects and disconnects the pulser 551 and the converting unit 605 to the 2D transducer array 640.

Source signals of the simultaneously transmitted type A codes and type B codes are reflected by a target object and are received by the 2D transducer array 640. In particular, the reflected codes which are received by the 2D transducer array 640 include both of the type A codes and type B codes. The 2D transducer array 640 converts the signals, which include both of the type A codes and type B codes, to electrical signals via transducers of the 2D transducer array 640, and output a plurality of electrical signals to the converting unit 305 via the T/R switch 304. Further, a number of the electrical signals is identical to the total number of transducers.

The reception unit 620 obtains codes which are transmitted by the transducer array 1011 in the elevation direction from signals that are reflected by a target object and received by the transducer array 1012 in the lateral direction, and obtains codes which are transmitted by the transducer array 1012 in the lateral direction from signals that are reflected by a target object and received by the transducer array 1011 in the elevation direction.

The converting unit 605 amplifies the reflected signals which are supplied by the 2D transducer array 640 via the T/R switch 604, and converts the amplified reflected signals into digital signals. For example, the converting unit 605 may include a pre-amplifier, a time gain compensation (TGC) unit for compensating for reductions in amplitude incurred by ultrasound waves due to the propagation of the ultrasound waves propagate inside a body, and an analog-to-digital converter (ADC).

The reception signal delaying unit 606 provides beamforming based on a delay look-up table to the reception beam-forming unit 607. The delay look-up table refers to information which relates to periods of time which correspond to respective differences in distances between the transducers and the focal point 20, which are stored in the form of a table and which are usable by the reception beam-forming unit 607 in order to merge electrical signals output by transducers into one signal.

When the reception beam-forming unit 607 receives the delay look-up table from the reception signal delaying unit 606, the reception beam-forming unit 607 delays electrical signals which have been converted by the converting unit 605 based on the delay look-up table, merges the electrical signals from predetermined transducers of the 2D transducer array 640 when all of the predetermined transducers of the transducer array 604 finish outputting the electrical signals, and outputs a merged signal to the reception decoder 609.

The reception decoder 609 receives signals which have been output by the reception beam-forming unit 607, separates the received signals into signals which include type A codes and signals which include type B codes, obtains image data from each of the separated signals, and outputs the image data to the image processor 610. The reason for separating the codes is that if a transducer in the 2D transducer array 640 array transmits a source signal for forming a 3D image, the transducer shall not receive the signal reflected by a target object, and another transducer array shall receive the reflected signal. Therefore, as described above in relation to the cross-transducer array, the transducer array 1012 in the lateral direction receives reflected signals which include type A codes if the transducer array 1011 in the elevation direction transmits type A codes, whereas the transducer array 1011 in the elevation direction receives reflected signals which include type B codes if the transducer array 1012 in the lateral direction transmits type B codes. It is necessary for the reception decoder 609 to perform a correlation process upon electrical signals which are received by the transducer array 1012 in the lateral direction with type A codes and obtain type A signals only, and to perform a correlation process on electrical signals which are received by the transducer array 1011 in the elevation direction with type B codes and obtain type B signals only.

FIGS. 7A, 7B, 7C, and 7D are diagrams which illustrate 2D transducer arrays, according to exemplary embodiments.

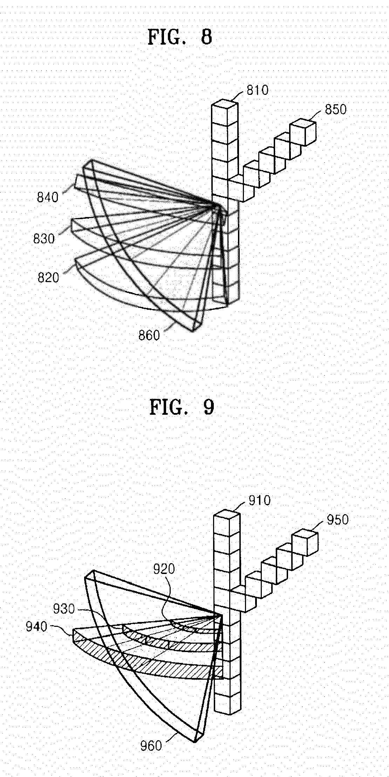

First, in a 2D transducer array 700a shown in FIG. 7A, six transducers are arranged in a lateral direction (shown as direction j), whereas six transducers are arranged in an elevation direction (shown as direction i). Therefore, the 2D transducer array 700a has a 6x6 matrix-like structure which includes a total of 36 transducers. In particular, the 2D transducer array 700a is provided to exemplify a MxN 2D transducer array, where values of both of M and N are equal to 6. The ultrasound volume scanning device 102 according to the present exemplary embodiment applies codes that are orthogonal to each other to transducers which are included in the 2D transducer array 700a. The ultrasound volume scanning device 102 applies codes that are orthogonal to each other to at least one 1D transducer array which is included in the 2D transducer array 700a. Detailed description regarding application of codes that are orthogonal to each other by the ultrasound volume scanning device 102 will be provided below with reference to FIG. 8. In particular, a 1D transducer array to which orthogonal codes are applied will be described. The 2D transducer array 700a includes a plurality of 1D transducer arrays. For example, when the 2D transducer array 700a is indicated as a matrix, each row and column corresponds to a respective 1D transducer array. Therefore, the 2D transducer array 700a includes a total of 12 1D transducer arrays. Transducers included in each of 1D transducer arrays which respectively correspond to rows and columns may be linearly arranged, and it is not necessary to arrange exactly six transducers in each of the 1D transducer array. For example, if each of transducers is indicated as (i, j), a group of five transducers which includes (0, 1), (0, 2), (0, 3), (0, 4), and (0, 5) may form a single 1D transducer array. Furthermore, transducers in a 1D transducer array may be linearly arranged and may not be parallel to a lateral direction or an elevation direction (see, for example, FIG. 7B). For example, a group of transducers (0, 0), (1, 1), (2, 2), (3, 3), (4, 4), and (5, 5) which is arranged in a diagonal direction forms another 1D transducer array, as illustrated, for example, in FIG. 7C. The ultrasound volume scanning device 102 uses at least one 1D transducer array which is included in a 2D transducer array as an array for transmitting ultrasound signals. As described above, it is necessary to arrange a 1D transducer array for receiving ultrasound signals in a direction which is different from the direction in which the 1D transducer array for transmitting the ultrasound signals is arranged, according to the present exemplary embodiment. Therefore, as shown in FIG. 7A, a 2D transducer array according to the present exemplary embodiment includes at least one transmission array and at least one reception array. In particular, it is necessary for a 2D transducer array according to the present exemplary embodiment to include at least two 1D transducer arrays. Further, although the ultrasound volume scanning device 102 may transmit and receive ultrasound signals by using all of the 36 transducers included in the 2D transducer array 700a, the ultrasound volume scanning device 102 may also transmit and receive ultrasound signals without using all of the 36 transducers included in the 2D transducer array 700a, and rather may do so just by using at least two 1D transducer arrays, as described above. Therefore, the ultrasound volume scanning device 102 according to the present exemplary embodiment may use a 2D transducer array which corresponds to the 2D transducer array 700a from which transducers not used for transmission and reception of ultrasound signals are removed.

The above descriptions are provided under an assumption that transducers in each of linear 1D transducer arrays which are included in the 2D transducer array 700a are arranged in a straight line. However, the definition of the term linear is not limited to a straight line herein, and may include, for example, a curved line. Furthermore, a modification may be made to the present exemplary embodiment to form a closed-curve, that is, an annular array by increasing curvature of a 1D transducer array, according to the present exemplary embodiment.

FIGS. 7B, 7C, and 7D show 2D transducer arrays from which transducers not used for transmission and reception of ultrasound signals are removed, according to exemplary embodiments. However, the 2D transducer arrays shown in FIGS. 7B, 7C, and 7D are merely examples of 2D transducer arrays which include at least two 1D transducer arrays each, and the present inventive concept is not limited thereto.

A method by which the ultrasound volume scanning device 102 applies a plurality of orthogonal codes to a single 1D transducer array which is included in the 2D transducer array 640 will be described below with reference to FIGS. 8, 9, 10, 11, and 12. In particular, a method by which the ultrasound volume scanning device 102 applies M orthogonal codes (wherein M>N) to N 1D transducer array(s) (wherein N is 1 or greater) which are included in the 2D transducer array 640 will be described.

FIG. 8 is a diagram which illustrates that a 1D transducer array 810 which is included in a 2D transducer array, according to an exemplary embodiment, forms a beam plane.

Referring to FIG. 8, the 1D transducer array 810 simultaneously forms three transmission beam planes 820, 830, and 840 with a single transmission. The transducer array 810 shown in FIG. 8 may be any of 1D transducer arrays which are included in the 2D transducer arrays as shown in FIGS. 5 and 7A through 7D.

In the related art, the 1D transducer array 810 sequentially forms the transmission beam planes 820, 830, and 840 by steering angles of focused ultrasound beams. However, according to the present exemplary embodiment, the transmission beam planes 820, 830, and 840 may be simultaneously formed by applying transmission delay patterns which correspond to respective steering angles of the transmission beam planes to codes that are orthogonal to each other. If it is assumed that N scan lines are formed in a single transmission beam plane, N scan lines may be formed via a single beam transmission in the related art, whereas 3*N scan lines may be formed via a single beam transmission according to the present exemplary embodiment. Although FIG. 8 shows the three beam planes 820, 830, and 840, it is merely an example for convenience of explanation, and K (wherein K>1) beam planes may be formed.

To distinguish the transmission beam planes 820, 830, and 840 from each other, the code outputting unit 611 outputs a code set A which includes codes that are orthogonal to each other to transmission beam-forming unit 603. The code set A includes codes a1, a2, and a3 that are orthogonal to each other. In particular, being orthogonal indicates that auto-correlation is equal to one and cross-correlation is equal to zero. Therefore, correlation between example codes ai and aj (here, i≠j) is zero. More particularly, being orthogonal indicates a state or a property in which two or more signal systems with the same properties may operate without interfering with each other. By using orthogonal codes, reflected signals may be analyzed without interference from other signals, even if two or more signals are simultaneously transmitted and subsequently reflected.

However, codes having a pseudo-orthogonal property may be used as orthogonal codes. The pseudo-orthogonal property indicates that a result of auto-correlation is similar to an impulse function (an "impulse function," as used herein, generally refers to a Dirac-delta function or a function which has properties including $\delta(t)=1$ at $t=0$ and $\delta(t)=0$ at $t\neq0$) and a result of cross-correlation is nearly equal to zero. In particular, being similar indicates that results of auto-correlation and cross-correlation at $t\neq0$ are smaller than 30 dB, for example, as compared to a result of correlation at $t=0$.

Hereinafter, a method for forming the transmission beam planes 820, 830, and 840 by using the orthogonal code set A will be described. The 1D transducer array 810 includes M transducers.

A first transmission delay pattern Z1 regarding the transmission beam plane 820 defines respective delay times regarding M transducers. In the same regard, there are a second transmission delay pattern Z2 and a third transmission delay pattern Z3 which respectively correspond to the transmission beam plane 830 and the transmission beam plane 840. The transmission signal delaying unit 602 receives inputs of steering angles $\Phi$ and focal distances r for the respective transmission beam planes 820, 830, and 840 from a control unit (not shown) and outputs the respective transmission delay patterns Z1, Z2, and Z3 to the transmission beam-forming unit 603.

[Equation 1]

$$Z1 = [d1, d2, d3, \ldots, dm]^{T}$$

$$Z2 = [e1, f2, f3, \ldots, fm]^{T}$$

$$Z3 = [f1, f2, f3, \ldots, fm]^{T}$$

The transmission beam-forming unit 603 receives the transmission delay patterns Z1, Z2, and Z3 from the transmission signal delaying unit 602 and delays a transmission signal g(t). In particular, signals generated by delaying the transmission signal g(t) based on transmission delay patterns may be expressed as a matrix G as shown in Equation 2 below.

[Equation 2]

$$\text{Matrix } G = \begin{bmatrix} g(t-d1) & g(t-e1) & g(t-f1) \\ g(t-d2) & g(t-e2) & g(t-f2) \\ g(t-d3) & g(t-e3) & g(t-f3) \\ g(t-d4) & g(t-e4) & g(t-f4) \\ \cdots & \cdots & \cdots \\ g(t-dm) & g(t-em) & g(t-fm) \end{bmatrix}$$

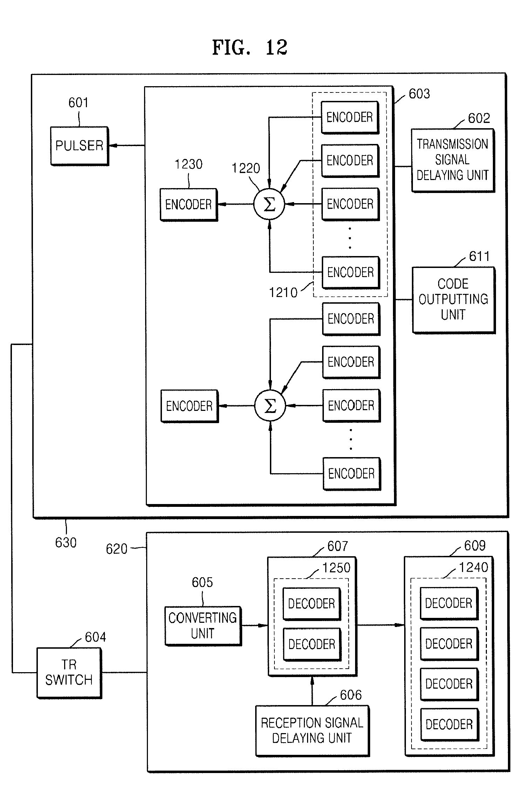

The $i^{th}$ transducer in the transducer array 810 corresponds to the $i^{th}$ row of the matrix G, whereas the transmission beam planes 820, 830, and 840 respectively correspond to columns of the matrix G. The signals in the matrix G are generated by time-delaying the same signal g(t), thus being correlated to each other. Therefore, if the signals in the matrix G are applied to the transducer array 810 without any further process, components of signals reflected by a target object may not be separated based on transmission beam planes. Therefore, according to the present exemplary embodiment, the orthogonal code set A and elements of the matrix G are convolved. The convolved elements are combined and applied to the transducer array 810. In particular, the transmission beam plane 603 convolves the delayed transmission signal matrix G with the orthogonal code set A. Further, the transmission beam-forming unit 603 includes an encoder for delaying the transmission signal g(t) and convolving delayed transmission signals with orthogonal codes. Detailed description of the transmission beam-forming unit 603 including the encoder will be provided below with reference to FIG. 12.

[Equation 3]

$$G * A$$
$$= \begin{bmatrix} a1 * g(t-d1) + a2 * g(t-e1) + a3 * g(t-f1) \\ a1 * g(t-d2) + a2 * g(t-e2) + a3 * g(t-f2) \\ a1 * g(t-d3) + a2 * g(t-e3) + a3 * g(t-f3) \\ \vdots \\ a1 * g(t-dm) + a2 * g(t-em) + a3 * g(t-fm) \end{bmatrix}$$

The operator "*" indicates convolution. Each of rows of the matrix shown in Equation 3 indicates a signal which is applied to each transducer of the transducer array 810.

Because signals which are respectively transmitted by transducers of the transducer array 810 are overlapped at the focal distance r of the transmission beam plane 820, beamforming is performed, and the signals become M X a1*g(t-q1). In particular, M indicates an amplitude of a signal at the focal distance r under an assumption that there is no signal loss due to a medium during propagation of transmission signals transmitted by M transducers. q1 represents a sum of propagation delay times of the transmission signals which are transmitted by M transducers and delay times of the transmission delay pattern Z1, and has the same value at the focal distance r with respect to all transducers. For example, it is assumed that a propagation delay time for a transmission signal from a first transducer to arrive at the transmission beam plane 820 is t1, and that a propagation delay time for a transmission signal from a $M^{th}$ transducer to arrive at the transmission beam plane 820 is tm. In this case, t1+d1=t2+d2=...= tm+dm, and thus ultrasound signals which are transmitted by respective transducers may be focused on the transmission beam plane 820 at the same time point. In the same regard, signals at the focal distance r of the transmission beam plane 830 become M X a2*g(t-q2), and signals at the focal distance r of the transmission beam plane 840 become M X a3*g(t-q3).

Hereinafter, a method for forming a reception beam plane by receiving signals which are reflected with respect to ultrasound beams which are focused on the transmission beam planes 820, 830, and 840 will be described. A transducer array 850 receives ultrasound signals which are reflected by the respective transmission beam planes 820, 830, and 840. The transducer array 850 is arranged in a direction which is different from the direction in which the transducer array 810 is arranged for forming the transmission beam planes 820, 830, and 840. For example, in any of the 2D transducer arrays shown in FIGS. 5 and 7A through 7D, when two 1D transducer arrays which are arranged in different directions on the same plane are selected, one of the two 1D transducer arrays is referred to as the transducer array 810, and the other one of the two 1D transducer arrays is referred to as the transducer array 850.

Reflected signals which are expressible as M X a1*g(t-q1), M X a2*g(t-q2), and M X a3*g(t-q3) arrive at the transducer array 850. When ultrasound signals are focused on a target object, signals are not totally reflected and are partially absorbed by the target object, and thus amplitudes of reflected signals are reduced. For convenience of explanation, it is assumed that amplitudes of reflected signals are reduced to 1/M. The transducer array 850 forms a reception beam plane 860 which has a focal distance r. As the reception beam plane 860 crosses the transmission beam planes 820, 830, and 840, three scan lines are formed. A reception delay pattern Z4 for forming the reception beam plane 860 which has a steering angle θ may be expressed as shown in Equation 4 below. When a steering angle θ and the focal distance r of the reception beam plane 860 are input by a control unit (not shown), the reception signal delaying unit 606 outputs the reception delay pattern Z4 regarding the reception beam plane 860 to the reception beam-forming unit 607.

[Equation 4]

$$Z4 = [w1, w2, w3, \ldots, wm]^T$$

The reception beam-forming unit 607 delays signals which are received by the respective transducers of the transducer array 850 based on the reception delay pattern Z4 input by the reception signal delaying unit 606. A result of applying the reception delay pattern Z4 to the signals which are received by the respective transducers of the transducer array 850 may be expressed as a matrix R as shown in Equation 5.

[Equation 5]

Matrix R

$$= \begin{bmatrix} a1*g(t-q1-u1-w1) + a2*g(t-q2-u1-w1) + a3*g(t-q3-u1-w1) \\ a1*g(t-q1-u2-w2) + a2*g(t-q2-u2-w2) + a3*g(t-q3-u2-w2) \\ a1*g(t-q1-u3-w3) + a2*g(t-q2-u3-w3) + a3*g(t-q3-u3-w3) \\ a1*g(t-q1-u4-w4) + a2*g(t-q2-u4-w4) + a3*g(t-q3-u4-w4) \\ a1*g(t-q1-u5-w5) + a2*g(t-q2-u5-w5) + a3*g(t-q3-u5-w5) \\ \vdots \\ a1*g(t-q1-um-wm) + a2*g(t-q2-um-wm) + a3*g(t-q3-u1-wm) \end{bmatrix}$$

Rows of the matrix R respectively indicate ultrasound signals which are received by the respective transducers of the transducer array 850 and delayed by applying the reception delay pattern Z4. In particular, u1 through um indicate propagation delays until respective ultrasound signals transmitted from the transmission beam plane 820 arrive at respective transducer elements of the transducer array 850. The reception beam-forming unit 607 performs reception beam formation by combining all elements of the matrix R to which the reception delay pattern Z4 is applied. In particular, in order to simultaneously form three scan lines, it is necessary to separate combined signals for each of the transmission beam planes 820, 830, and 840.

The reception decoder 609 uses the orthogonal code set A which is received from the code outputting unit 611 in order to separate the combined signals of the matrix R for each of the transmission beam planes 820, 830, and 840. Hereinafter, a method for separating signals for the transmission beam plane 820 will be described. The reception decoder 609 performs correlation between the combined signals and a code a1. Due to the orthogonal property of the code set A, polynomial terms which are convolvod with code a2 or code a3 are eliminated from the combined signals as a result of the correlation. Therefore, only terms which are convolved with code a1 remain. When all of the remaining terms are combined, image information regarding scan lines formed by the transmission beam plane 820 and the reception beam plane 860 may be obtained. A result thereof may be expressed as shown in Equation 6.

[Equation 6]

$$R1 = g(t-q1-u1-w1) + g(t-q1-u1-w2) + g(t-q1-u1-w3) + ..... + g(t-q1-u1-wm)$$

In Equation 6, q1+u1+w1 = q1+u1+w2 = ... = q1+u1+wm, because the reception delay pattern Z4 compensates propagation delays which have occurred as a result of reception. Therefore, signals received by the respective transducers of the transducer array 850 may be focused as a single ultrasound signal.

In the same regard, image information may be obtained from scan lines which are formed by the transmission beam planes 830 and 840 and the reception beam plane 860 by applying the same method described above to codes a2 and a3 of the code set A. However, although correlation with the code set A is performed above after all elements of the matrix R are combined, an identical result may be obtained by performing correlations and combining results of the correlations. The codes a1, a2, and a3 of the code set A may be sequentially applied. Alternatively, correlations with the codes a1, a2, and a3 may be performed in parallel by arranging a plurality of reception decoders in parallel. In this case, three scan lines may be simultaneously formed. A structure of the reception unit 620 in which a plurality of reception decoders are arranged in parallel will be described below with reference to FIG. 12.

Further, the formation of scan lines as described above may also be applied to other reception beam planes which have steering angles $\Theta$ which are different from that of the reception beam plane 860 in the same regard. In this case, it is necessary to apply reception delay patterns Z5, Z6, and so on in correspondence to the steering angles $\Theta$, instead of the reception delay pattern Z4. A plurality of reception beam planes may be simultaneously formed by applying reception delay patterns in parallel. If s transmission beam planes are simultaneously formed and t reception beam planes are simultaneously formed, image information may be obtained from s X t scan lines. In particular, image information may be obtained from s X t scan lines by performing single ultrasound transmission and single ultrasound reception, and thus a period of time which is required for forming a 3D image may be reduced. Furthermore, the values s and t may be suitably adjusted to obtain 3D images in real time. For example, higher s and t values are needed for 3D images of 30 frames/sec, as compared to 3D images of 10 frames/sec.

FIG. 9 is a diagram which illustrates dynamic beam focusing according to an exemplary embodiment. As described above, although the dynamic beam focusing is different from the steering of an ultrasound beam, the dynamic beam focusing is based on the same mechanism as the steering of an ultrasound beam. Therefore, descriptions similar to those given above with reference to FIG. 8 will be omitted.

Referring to FIG. 9, a 1D transducer array 910 focuses ultrasound beams to three focal distances 920, 930, and 940 via a single transmission. The 1D transducer array 910 of FIG. 9 is any of 1D transducer arrays included in the 2D transducer arrays as shown in FIGS. 5 and 7A through 7D.

Ultrasound beams may be focused at the plurality of focal distances 920, 930, and 940 by applying reception delay patterns which respectively correspond to the focal distances 920, 930, and 940 to codes which are orthogonal to each other. In the related art, a number of ultrasound beam transmissions increases in proportion to a number of focal distances for dynamic focusing during transmitting ultrasound signals. Therefore, in the related art, a period of time which is required for obtaining a 3D image increases, and thus it is difficult to obtain 3D images in real time. However, according to the present exemplary embodiment, dynamic focusing of ultrasound beams may be performed with a single transmission of ultrasound signals, and thus a 3D image with improved resolution in the elevation direction may be obtained without increasing a period of time which is required for obtaining the 3D image. Although FIG. 9 shows that ultrasound beams are focused at three different focal distances, it is merely an example for convenience of explanation, and ultrasound beams may be focused at K (wherein K>1) focal distances.

The code outputting unit 611 outputs code set B, which includes codes that are orthogonal to each other, to the reception beam-forming unit 607 in order to distinguish ultrasound beams which are respectively focused at the focal distances 920, 930, and 940. The code set B={b1, b2, b3} includes codes b1, b2, and b3 that are orthogonal to each other. The 1D transducer array includes M transducers.

A first transmission delay pattern Y1 defines respective delay times for the M transducers with respect to the focal distances 920. In the same regard, there are a second transmission delay pattern Y2 and a third transmission delay pattern Y3 which respectively correspond to the focal distance 930 and the focal distance 940. The transmission signal delaying unit 602 receives inputs of r values of the focal distances 920, 930, and 940 from a control unit (not shown) and outputs the transmission delay patterns Y1, Y2, and Y3 to the reception beam-forming unit 607.

[Equation 7]

$$Y1 = [h1, h2, h3, \ldots\ldots, hm]^T$$

$$Y2 = [i1, i2, i3, \ldots\ldots, im]^T$$

$$Y3 = [j1, j2, j3, \ldots\ldots, jm]^T$$

The transmission beam-forming unit 603 delays the transmission signal g(t) based on the transmission delay patterns Y1, Y2, and Y3. Signals which are generated as the transmission beam-forming unit 603 delays the transmission signal g(t) based on the transmission delay patterns Y1, Y2, and Y3 may be expressed as a matrix G as shown in Equation 8 below.

[Equation 8]

$$\text{Matrix } G = \begin{bmatrix} g(t-h1) & g(t-i1) & g(t-j1) \\ g(t-h2) & g(t-i2) & g(t-j2) \\ g(t-h3) & g(t-i3) & g(t-j3) \\ g(t-h4) & g(t-i4) & g(t-j4) \\ \ldots & \ldots & \ldots \\ g(t-hm) & g(t-im) & g(t-jm) \end{bmatrix}$$

Similar to the direction of transmission beam as described above, the transmission beam-forming unit 603 convolves the orthogonal code set B with elements of the matrix G and combines the convolved elements. The combined signal is applied to the transducer array 910 via the T/R switch 604.

[Equation 9]

$$G * B = \begin{bmatrix} b1 * g(t-h1) + b2 * g(t-i1) + b3 * g(t-j1) \\ b1 * g(t-h2) + b2 * g(t-i2) + b3 * g(t-j2) \\ b1 * g(t-h3) + b2 * g(t-i3) + b3 * g(t-j3) \\ \cdot \\ \cdot \\ \cdot \\ b1 * g(t-hm) + b2 * g(t-im) + b3 * g(t-jm) \end{bmatrix}$$

Rows of the matrix shown in Equation 9 respectively indicate signals applied to respective transducers of the transducer array 910.

At each of the focal distances 920, 930, and 940, signals which are transmitted by the respective transducers of the transducer array 910 are overlapped and become M X b1*g(t-q1). q1 represents a sum of propagation delay times of the transmission signals which are transmitted by M transducers and delay times of the transmission delay pattern Y1. In the same regard, signals at the focal distance 930 become M X b2*g(t-q2), and signals at the focal distance 940 become M X b3*g(t-q3).

Hereinafter, a method for receiving signals which are reflected by the respective focal distances 920, 930, and 940 and performing dynamic reception focusing will be described. A transducer array 950 receives ultrasound signals which are reflected by the respective focal distances 920, 930, and 940. The transducer array 950 is arranged in a direction which is different from the transducer array 910. For example, when two 1D transducer arrays which are arranged in different directions are selected from any of the 2D transducer arrays shown in FIGS. 5 and 7A through 7D, one of the selected 1D transducer arrays becomes the transducer array 910, whereas the other one becomes the transducer array 950.

Reflected signals which are expressible as M X a1*g(t-q1), M X a2*g(t-q2), and M X a3*g(t-q3) arrive at the transducer array 950. When ultrasound signals are actually reflected by a target object, signals are not totally reflected and are partially absorbed by the target object. For convenience of explanation, it is assumed that amplitudes of reflected signals are reduced to 1/M.

The signals received by the respective transducers of the transducer array 950 may be expressed as a matrix R as shown in Equation 10 below.

[Equation 10]

Matrix R

$$= \begin{bmatrix} b1 * g(t-q1-u1) + b2 * g(t-q2-v1) + b3 * g(t-q3-t1) \\ b1 * g(t-q1-u2) + b2 * g(t-q2-v2) + b3 * g(t-q3-t2) \\ b1 * g(t-q1-u3) + b2 * g(t-q2-v3) + b3 * g(t-q3-t3) \\ \cdot \\ \cdot \\ \cdot \\ b1 * g(t-q1-um) + b2 * g(t-q2-vm) + b3 * g(t-q3-tm) \end{bmatrix}$$

At an $i^{th}$ row, ui, vi, and ti indicate propagation delays until the ultrasound signals reflected with respect to the respective focal distances 920, 930, and 940 arrive at respective components of the transducer array 850. The code outputting unit 611 outputs the orthogonal code set B to the reception decoder 609 in order to separate the signals which correspond to the matrix R for each of the focal distances 920, 930, and 940, Hereinafter, a method for separating signal components which correspond to the focal distance 920 will be described. The reception decoder 609 performs correlations between each row of the matrix R and the code b1. As a result, elements of the $i^{th}$ row may be expressed as shown in Equation 11 below.

[Equation 11]

$$Ri(t) = g(t - q1 - ui)$$

For the transducer array 950 to perform reception beam formation with respect to a location which corresponds to the focal distance r, a control unit (not shown) provides a value of the focal distance r to the reception signal delaying unit 606. The reception signal delaying unit 606 outputs a reception delay pattern Y4 to the reception beam-forming unit 607. The focal distance r may correspond to an arbitrary location in the depth-wise direction. However, for convenience of explanation, it is assumed that the focal distance r corresponds to the same location as the focal distance 920. The reception delay pattern Y4 corresponding to the focal distance r may be expressed as shown in Equation 12 below.

[Equation 12]

$$Y4 = [x1, x2, x3, \ldots, xm]^T$$

The reception delay pattern Y4 compensates propagation delay times of reflected signals. In particular, a reception beam may be focused by applying the reception delay pattern Y4 to signals which are reflected with respect to the focal distances 920. More particularly, u1 + x1 = u2 + x2 = ... = um + xm. The reception beam-forming unit 607 combines reflected signals by applying the reception delay pattern Y4. A result thereof may be expressed as shown in Equation 13 below.

[Equation 13]

$$R1(t) = g(t - q1 - u1 - x1) + g(t - q1 - u2 - x1) + \ldots + g(t - q1 - um - x1)$$

$$= M^* g(t - p1)$$

In the same regard, dynamic reception focusing regarding the focal distances 930 and 940 may be performed by applying the same method described above with respect to the codes b2 and b3 of the code set B. The codes b1, b2, and b3 of the code set B may be sequentially applied. Alternatively, correlations with the codes b1, b2, and b3 may be performed in parallel by arranging a plurality of correlation calculators in parallel. In this case, the reception decoder 609 includes a plurality of decoders that are arranged in parallel in order to perform correlations of a plurality of codes in parallel. A structure of the reception decoder 609 will be described below with reference to FIG. 12.

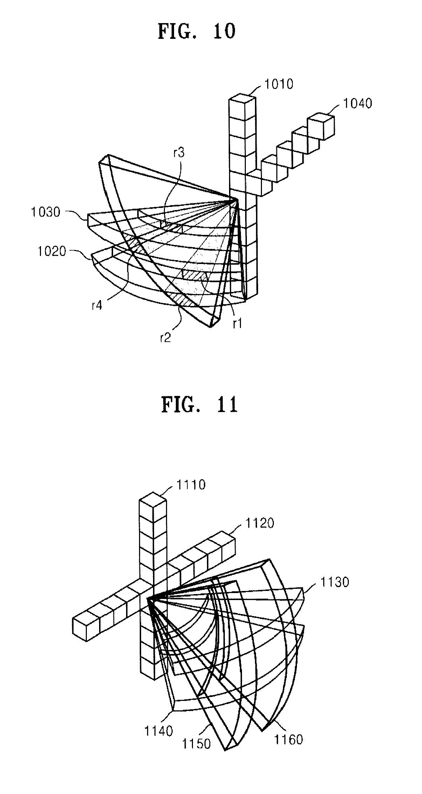

FIG. 10 is a diagram which illustrates steering and dynamic focusing of ultrasound beams, according to an exemplary embodiment.

Referring to FIG. 10, a method for transmitting and receiving ultrasound beams by simultaneously applying the steering of ultrasound beam as shown in FIG. 8 and dynamic focusing of ultrasound beam as shown in FIG. 9 is illustrated. As described above, the dynamic focusing and steering of ultrasound beams are common for changing locations at which ultrasound signals are focused by using delay patterns. Furthermore, orthogonal codes which are different from one another are used in correspondence to the delay patterns, respectively, Therefore, performing steering and dynamic focusing of ultrasound beams as shown in FIG. 10 may be understood as focusing ultrasound beams by using delay patterns which are different from one another and by using orthogonal codes which respectively correspond to the delay patterns. Descriptions similar to those given above with reference to FIGS. 8 and 9 will be omitted below.

Referring to FIG. 10, a transducer array 1010 forms a first transmission beam plane 1020 and a second transmission beam plane 1030. A first focal distance r1 and a second focal distance r2 are located on the first transmission beam plane 1020, whereas a third focal distance r3 and a fourth focal distance r4 are located on the second transmission beam plane 1030. In particular, the transducer array 1010 performs two-way dynamic focusing of ultrasound beams at the two focal distances r1 and r2 in correspondence with steering angle $\Phi1$ of the first transmission beam plane 1020 and performs dynamic focusing of ultrasound beams at the two focal distances r3 and r4 in correspondence with steering angle $\Phi2$ of the second transmission beam plane 1030. According to the present exemplary embodiment, k (wherein k>1) transmission beam planes and l (wherein l>1) reception beam planes may be formed. However, for convenience of explanation, a method for forming two transmission beam planes and two reception beam planes will be described below.

First, the transmission delay patterns Z1, Z2, Z3, and Z4 may be expressed as shown in Equation 14 below. The transmission delay pattern Z1 corresponds to the steering angle $\Phi1$ and the first focal distance r1, the transmission delay pattern Z2 corresponds to the steering angle $\Phi1$ and the second focal distance r2, the transmission delay pattorn Z3 corresponds to the steering angle $\Phi2$ and the third focal distance r3, and the transmission delay pattern Z4 corresponds to the steering angle $\Phi2$ and the fourth focal distance r4. The transmission signal delaying unit 602 receives ($\Phi1$, r1), ($\Phi1$, r2), ($\Phi2$, r3), and ($\Phi2$, r4) from a control unit (not shown) and outputs the transmission delay patterns Z1, Z2, Z3, and Z4 to the transmission beam-forming unit 603.

[Equation 14]

$$Z1 = [a1, a2, a3, \ldots\ldots, am]^T$$

$$Z2 = [b1, b2, b3, \ldots\ldots, bm]^T$$

$$Z3 = [c1, c2, c3, \ldots\ldots, cm]^T$$

$$Z4 = [d1, d2, d3, \ldots\ldots, dm]^T$$

The code outputting unit 611 outputs a code set E={e1, e2, e3, e4} which includes codes that are orthogonal to each other to the reception beam-forming unit 607. The reception beam-forming unit 607 applies the orthogonal code set E to transmission signals. The transmission signals to which the code set E are applied may be expressed as shown in Equation 15 below. In Equation 15, Gi(t) indicates a signal applied to an ith transducer of the transducer array 1010.

[Equation 15]

$$Gi(t) = e1*(t-ai) + e2*(t-bi) + e3*(t-ci) + e4*(t-di)$$

A transducer array, 1040 receives signals which are reflected by a target object. Similarly as shown in FIGS. 8 and 9, the transducer array 1010 and the transducer array 1040 are 1D transducer arrays which are included in a 2D transducer array and are arranged in directions. A signal Ri(t) which is received by an ith transducer of the transducer array 1040 may be expressed as shown in Equation 16 below.

[Equation 16]

$$Ri(t)=e1*(t-q1-\xi)+e2*(t-q2-yi)+e3*(t-q3-wi)+e4*(t-q4-zi)$$

Hereinafter, a method for obtaining signal components which are reflected with respect to the first focal distance r1 of the first transmission beam plane 1020 from ultrasound signals received by the transducer array 1040 will be described. The reception decoder 609 may obtain $Ri(t)' = e1*(t-q1-xi)$ by removing signal components from $Ri(t)$ other than terms which are convolved with the code e1 from among the code set E.

In order for the transducer array 1040 to form a reception beam plane 150 which has a steering angle $\Theta$, the reception signal delaying unit 606 outputs a reception delay pattern Z5 which corresponds to the angle $\Theta$ and the first focal distance r1 to the reception beam-forming unit 607. The reception beam-forming unit 607 delays $Ri(t)'$ by using the reception delay pattern Z5 and combines delayed signals. As described above with reference to FIG. 8, a plurality of scan lines may be formed at the first focal distance r1 of the first transmission beam plane 1020 by changing the angle $\Theta$. In the same regard, the reception unit 620 may form a plurality of scan lines at each of the second focal distance r2 of the first transmission beam plane 1020 and the third and fourth focal distances r3 and r4 of the second transmission beam plane 1030.

FIG. 11 is a diagram which illustrates dynamic focusing of an ultrasound beam by using a plurality of 1D transducer arrays, according to an exemplary embodiment. Referring to FIG. 11, two 1D transducer arrays 1110 and 1120 are arranged in different directions. In particular, each of the 1D transducer arrays 1110 and 1120 performs two-way dynamic focusing of an ultrasound beam and forms a plurality of transmission beam planes, similarly as the transducer array 1010 of FIG. 10. Therefore, descriptions same as those given above with reference to FIGS. 8 through 10 will be omitted.

Each of the transducer arrays 1110 and 1120 not only transmits ultrasound signals, but also receives ultrasound signals which are reflected by a target object. Ultrasound signals which are reflected by transmission beam planes 1130 and 1140 formed by the transducer array 1110 are received by the transducer array 1120, whereas ultrasound signals which are reflected by transmission beam planes 1150 and 1160 formed by the transducer array 1120 are received by the transducer array 1110. Further, in order to distinguish ultrasound signals which are transmitted by the transducer array 1110 from ultrasound signals which are transmitted by the transducer array 1120, the code outputting unit 611 outputs a code set Y={y1, y2} which includes codes that are orthogonal to each other to the transmission beam-forming unit 603. In particular, signals which are applied to the transducer arrays 1110 and 1120 are signals which correspond to the Gi(t) signal shown in Equation 15 convolved with another orthogonal code set Y. More particularly, when a to be applied to the transducer array 1110 is Si(t) and a signal to be applied to the transducer array 1120 is Ti(t), Si(t) and Ti(t) may be expressed as shown in Equation 17 below

[Equation 17]

$$Si(t)=y1*Gi(t)$$

$$Ti(t)=y2*Gi(t)$$

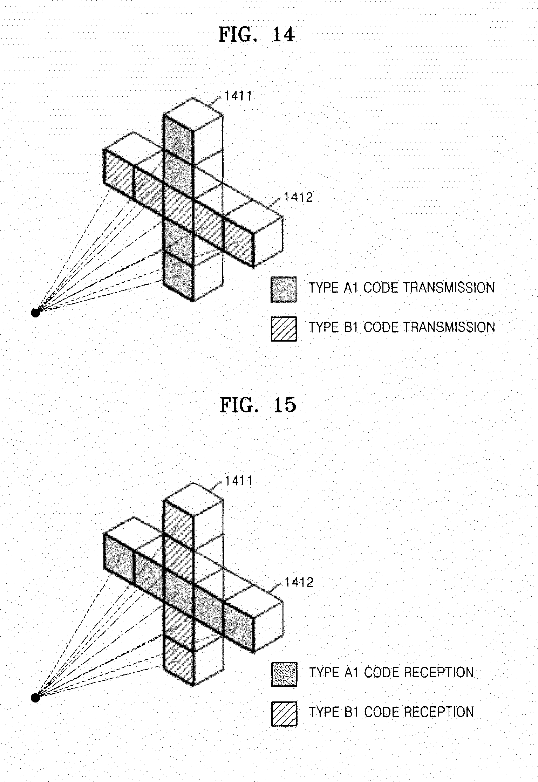

Equation 17 is set forth under an assumption that the transducer array 1110 uses Gi(t) as shown in Equation 15. Although it will be obvious to one of ordinary skill in the art that signals other than Gi(t) shown in FIG. 15 may be used by applying transmission delay patterns other than the transmission delay patterns shown in Equation 14, a case in which Gi(t) is used will be described below.

As described above with reference to FIG. 11, the transducer array 1110 performs dynamic focusing during transmission of ultrasound signals with respect to a plurality of transmission beam planes which correspond to Si(t), whereas the transducer array 1120 performs dynamic focusing during transmission of ultrasound signals with respect to a plurality of transmission beam planes which correspond to Ti(t).

Signals which are received by the transducer array 1110 may be expressed by using Ri(t) as shown in Equation 16. The signals which are received by the transducer array 1110 include not only reflected signals of ultrasound signals which are transmitted by the transducer array 1120, but also reflected signals of ultrasound signals which are transmitted by the transducer array 1110. A signal Qi(t) which is received by the transducer array 1110 may be expressed as shown in Equation 18 below.

[Equation 18]

$$Qi(t)=y1*Ri(t)+y2*Ri(t)$$

However, because the transducer array 1110 may not form a scan line with respect to a transmission beam plane formed by the transducer array 1110, it is necessary for the transducer array 1110 to form a scan line with respect to a transmission beam plane formed by the transducer array 1120. Therefore, it is necessary for the transducer array 1110 to remove signal components which correspond to the reflected signals of the ultrasound signals which are transmitted by the transducer array 1110. To this end, the reception decoder 609 performs correlation between the received signal Qi(t) and the orthogonal code y2 and removes terms which are convolved with the code y1. After the terms which are convolved with the code y1 are removed, dynamic reception focusing and steering of reception beam planes are performed in the same regard as described above with reference to FIG. 10. Furthermore, formation of a scan line by using reflected signals which are received by the transducer array 1120 is substantially the same as the formation of a scan line by the transducer array 1110 except for usage of the orthogonal code y1, and thus detailed description thereof will be omitted. Although FIG. 11 shows that two 1D transducer arrays are used, the method described above may be applied to K (wherein K>1) 1D transducer arrays by using K orthogonal codes.

FIG. 12 is a diagram which illustrates structures of the transmitter 630 and the receiver 620, according to an exemplary embodiment. The transmitter 630 and the receiver 620 shown in FIG. 12 have structures for one 1D transducer array which is included in a 2D transducer array in order to simultaneously form a plurality of beam planes or to perform dynamic focusing by using orthogonal codes, as described above with reference to FIGS. 8, 9, 10, and 11. The transmitter 630 includes the pulser 601, the transmission beam-forming unit 603, and the code outputting unit 611. Descriptions of the pulser 601 and the code outputting unit 611 are provided above.

The transmission beam-forming unit 603 includes N encoders 1210 and a combining unit 1220. The N encoders 1210 are arranged in parallel, and the combining unit 1210 combines signals which are output by the respective encoders 1210. The N encoders 1210 receive N transmission delay patterns from the transmission signal delaying unit 602 and receive N codes that are orthogonal to each other from the code outputting unit 611. In the case where N=3 as shown in FIG. 8, each encoder receives a transmission delay pattern and an orthogonal code regarding one transmission beam plane. Each encoder delays a transmission signal g(t) based on the transmission delay pattern and performs convolution with the orthogonal code. For example, from among the N encoders 1210, a first encoder receives a transmission delay pattern Z1 which relates to the transmission beam plane 820 from the transmission signal delaying unit 602 and receives the code a1 from among the orthogonal code set A from the code outputting unit 611. The first encoder delays the transmission signal g(t) based on the transmission delay pattern Z1 and convolves the same with the code a1. In the same regard, a second encoder and a third encoder encode the transmission signal g(t) with respect to the transmission beam plane 830 and the transmission beam plane 840, respectively.

The combining unit 1220 combines signals which are received from the N encoders 1210. When the N encoders 1210 encode and output the transmission signal g(t), the combining unit 1220 combines the output transmission signals and outputs signals which respectively correspond to rows of a matrix as shown in Equation 3. The signal combined by the combining unit 1220 is applied to the 2D transducer array 640 via the pulser 601 and the T/R switch 604. Although the case shown in FIG. 8 is provided as an example in the above description, the description may also be applied to the transmission delay patterns and the orthogonal codes shown in FIGS. 9 and 10 in the same regard. In particular, FIG. 10 provides the four transmission delay patterns Z1, Z2, Z3, and Z4 and the orthogonal code set E, which includes four codes that are orthogonal to each other. Therefore, the transmission beam-forming unit 603 shown in FIG. 10 has a structure in which four encoders are arranged in parallel.

Comparing FIG. 11 to FIG. 10, the transmission beam-forming unit 603 of FIG. 11 uses the orthogonal code set E of FIG. 10 and also uses the orthogonal code set Y to distinguish the transducer arrays 1110 and 1120. In particular, the transmission beam-forming unit 603 of FIG. 11 uses two orthogonal code sets, that is, the orthogonal code set E and the orthogonal code set Y. The N encoders 1210 described above encode a transmission signal g(t), and the combining unit 1220 combines encoded signals by using the orthogonal code set E and outputs the signal Gi(t) of Equation 15. Meanwhile, in FIG. 11, an encoder 1230 is used to convolve the signal Gi(t) of Equation 15 with the orthogonal code set E in order to distinguish signals which are transmitted by the transducer arrays 1110 and 1120. In particular, the encoder 1230 encodes the signal Gi(t) which is received from the combining unit 1220 by using the code y1. A signal which is output by the encoder 1230 is applied to the transducer array 1110 via the pulser 601 and the T/R switch 604. In addition, it is necessary for a signal to be applied to the transducer array 1120 to be processed in the same manner as the signal applied to the transducer array 1110. The transmission beam-forming unit 603 may sequentially apply signals to each of the transducer array 1110 and the transducer array 1120. However, the transmission beam-forming unit 603 may also simultaneously apply signals to the transducer array 1110 and the transducer array 1120. In a case where the transmission beam-forming unit 603 simultaneously applies signals to the transducer array 1110 and the transducer array 1120, the N encoders 1210, the combining unit 1220, and the encoder 1230 operate with respect to the transducer array 1110. N encoders (not shown), a combining unit (not shown) and an encoder (not shown) may be arranged in parallel to the N encoders 1210, the combining unit 1220, and the encoder 1230 and operate with respect to the transducer array 1120.

The receiver 620 includes the converting unit 605, the reception signal delaying unit 606, the reception beam-forming unit 607, and the reception decoder 609. Detailed descriptions of the converting unit 605 and the reception signal delaying unit reception signal delaying unit 606 are as provided above with reference to FIG. 6.

The reception decoder 609 includes N decoders 1240 that are arranged in parallel. In particular, N is proportional to a number of used orthogonal codes. In FIG. 8, because there are three codes a1, a2, and a3 in the orthogonal code set, the reception decoder 609 includes three decoders 1240. Each of the decoders 1240 outputs reception signals of Equation 6 by convolving each of the rows of the matrix R of Equation 5 with the codes a1, a2, and a3. Although the above description is based on the case shown in FIG. 8, the same may be applied to the orthogonal codes in FIGS. 9 and 10. Further, because FIG. 10 shows the orthogonal code set E which includes four codes, the reception decoder 609 of FIG. 10 may have a configuration in which four decoders are arranged in parallel.

Further, FIG. 11 uses the two orthogonal code set, that is, the orthogonal code set E and the orthogonal code set Y. In particular, as described above, the reception decoder 600 convolves the orthogonal code set E with the Ri(t) of Equation 16. The receiver 620 of FIG. 12 includes a decoder 1250 for calculating a correlation between the Qi(t) of Equation 18 and the orthogonal code set Y before the reception decoder 609 calculates a correlation between Qi(t) of Equation 18 and the orthogonal code set E. FIG. 11 shows that the decoder 607 is included in the reception beam-forming unit 607, however the decoder 1250 may be arranged before the decoders 1240 for processing signals before the decoders 1240 do. The decoder 1250 may include a plurality of decoders for processing correlations with orthogonal codes y1 and y2 in parallel. In FIG. 11, the decoder 1250 includes two decoders arranged in parallel. The decoder 1250 outputs Ri(t) of Equation 16 to the reception decoder 609 by correlating Qi(t) of Equation 18 with the orthogonal code set Y.

An example of an orthogonal code may include a Golay code. The Golay code is one of error correction codes which is used in digital communication and is a set of complementary bi-phase sequences. From among bi-phase codes, as known in the art, the Golay code features complete removal of a side lobe from a pulse-compressed output. Therefore, there have been many prior attempts to apply the Golay code to ultrasound imaging devices which use long pulses. For example, it is assumed that, in the Golay code, type A codes and type B codes are orthogonal to each other, where the type A codes include a code a1 and a code a2, whereas the type B codes include a code b1 and a code b2. There may be two or more codes per code type.

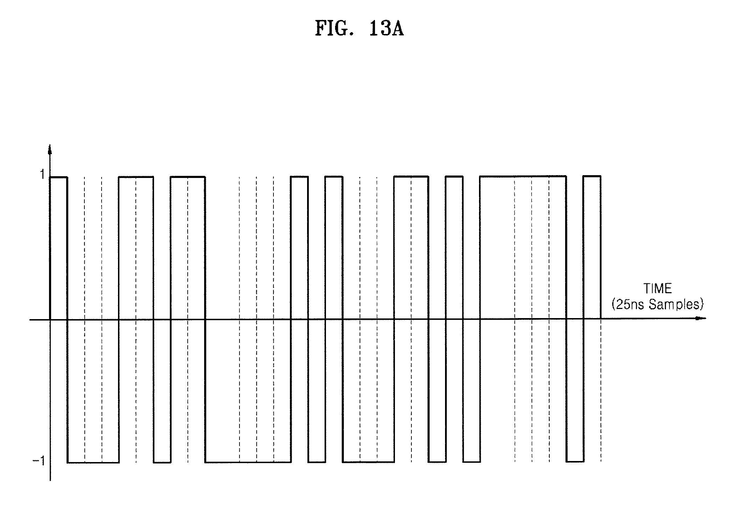

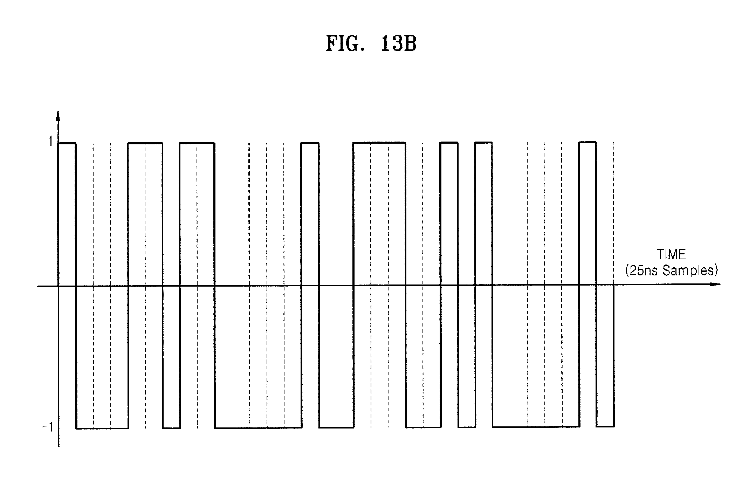

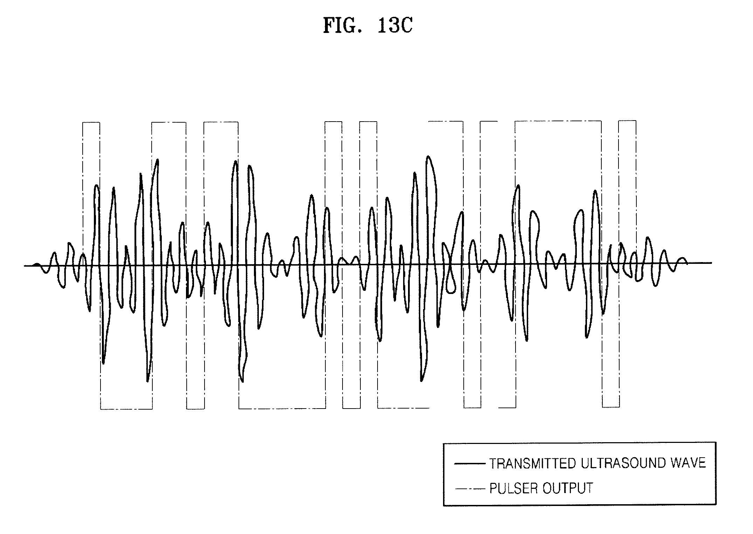

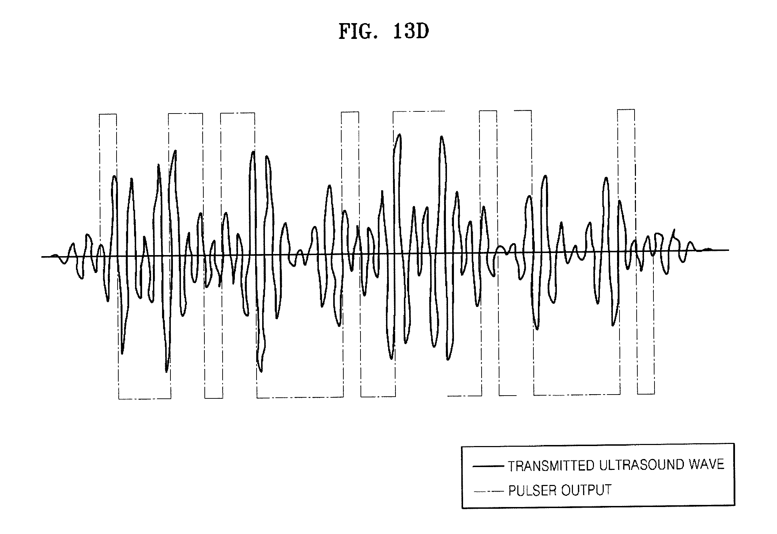

FIG. 13A is a diagram which illustrates an example of type A Golay codes. FIG. 13B is a diagram which illustrates an example of type B Golay codes. A code ai is shown in FIG. 13A, whereas a code bi is shown in FIG. 13B. The code ai is a code which has a sequence of {1,-1,-1,-1,1,1,-1,1,1,-1,-1,-1,-1,-1,1,-1,1,-1,-1,-1,1,1,-1,1,-1,1,1,1,1,1,-1,1}. The code bi is a code which has a sequence of {1,-1,-1,-1,1,1,-1,1,1,-1,-1,-1,-1,-1,1,-1,-1,1.1,1,-1,-1,1,1,-1,-t-1,-1,-1,1,-1}. FIG. 13C is a diagram which illustrates sound waves which are generated by a transducer when the code shown in FIG. 13A is output by the pulser 601. FIG. 13D is a diagram which illustrates sound waves which are generated by a transducer when the code shown in FIG. 13B is output by the pulser 601.

A method by which the ultrasound volume scanning device 102 applies a plurality of orthogonal codes to a single 1D transducer array which is included in a 2D transducer array is described above with reference to FIGS. 8 through 12. In particular, a method by which the ultrasound volume scanning device 102 applies M (wherein M>N) orthogonal codes to N 1D transducer array(s) (wherein N is one or greater) which are included in a 2D transducer array is described. However, the ultrasound volume scanning device 102 according to the present exemplary embodiment may apply at least two orthogonal codes to at least two 1D transducer arrays which are included in a 2D transducer array, so that only one orthogonal code may be applied to each 1D transducer array. Hereinafter, a method for applying N codes that are orthogonal to each other to a 2D transducer array in a case where N (wherein N>1) 1D transducer arrays are included in the 2D transducer array will be described. For convenience of explanation, it will be assumed that N=2.

FIG. 14 is a diagram which illustrates a 2D transducer array which is configured to transmit two types of code to a focus point. The 2D transducer array of FIG. 14 may be any of the 2D transducer arrays shown in FIGS. 5 and 7A through 7D. For convenience of explanation, it will be assumed that the 2D transducer array of FIG. 14 is the cross-transducer array of FIG. 5. Furthermore, it will be assumed that the orthogonal codes of FIG. 14 are the Golay codes shown in FIGS. 13A and 13B.

Although FIG. 14 shows that the transducer array 1411 in the elevation direction includes five transducers and the transducer array 1412 in the lateral direction includes five transducers (the center transducer is shared) for convenience of explanation, one of ordinary skill in the art will understand that the number of transducers may vary. The transducer array 1411 in the elevation direction (i.e., the y-axis direction) transmits the code a1, whereas the transducer array 1412 in the lateral direction (i.e., the x-axis direction) may transmit the code b1. Two types of code may be transmitted at the same time as described above, because, even if the two types of code propagate together and are reflected, the two types of code may be separated from each other due to their orthogonality. In particular, one orthogonal code is applied to one 1D transducer array in FIG. 14.

FIG. 15 is a diagram which illustrates the cross-transducer array 1411 and 1412 of FIG. 14 as being configured to receive two types of code. In particular, reflected signals which are incident to the transducer array 1411 in the elevation direction (i.e., the y-axis direction) include not only the cade b1, but also the cade a1. However, the transducer array 1411 in the elevation direction (i.e., the y-axis direction) only requires the reflected signal of the cade b1, not the reflected signal of the cade a1. The reason for this is that, as described above, it is necessary for a transducer array which is used for transmission and a transducer array which is used for reception to intersect each other perpendicularly. Therefore, the reception decoder 609 separates only the code b1 from signals which are incident to the transducer array 1411 in the elevation direction (i.e., the y-axis direction). In the same regard, the transducer array 1412 in the lateral direction (i.e., the x-axis direction) only requires the reflected signal of the code a1, and does not require the reflected signal of the code b1. Therefore, the reception decoder 609 separates only the code a1 from signals which are incident to the transducer array 1412 in the lateral direction (i.e., the x-axis direction). Therefore, although FIG. 15 shows that the transducer array 1411 in the elevation direction (i.e., the y-axis direction) receives only the code b1 and the transducer array 1412 in the lateral direction (i.e., the x-axis direction) receives only the code a1, the cross-transducer array of 1411 and 1412 actually receives both types of code, and FIG. 15 only shows codes which are separated and used by the reception decoder 609 after the reception of the codes. In this aspect, the transducer array 1411 in the elevation direction (i.e., the y-axis direction) separates only the code b1 from received reflected signals, whereas the transducer array 1412 in the lateral direction (i.e., the x-axis direction) separates only the code a1 from received reflected signals. As described above, the reception decoder 609 separates signals which are received by the reception decoder 609 based on the types of code.

Further, when the cross-transducer arrays 1411 and 1412 receive transmission signal patterns from the pulser 601 and transmit type A codes and type B codes, the cross-transducer arrays 1411 and 1412 may use a plurality of codes per each type of code transmitted. For example, the codes a1 and a2, which are type A codes, and the codes b1 and b2, which are type B codes, may be successively transmitted. In this aspect, the code a1 and the code b1 may be transmitted within a type A code and a type B code, respectively. After the codes a1 and b1 are reflected at a focus point and return, the codes a2 and b2, which are also respectively transmitted within the type A code and the type B code, may be transmitted immediately.

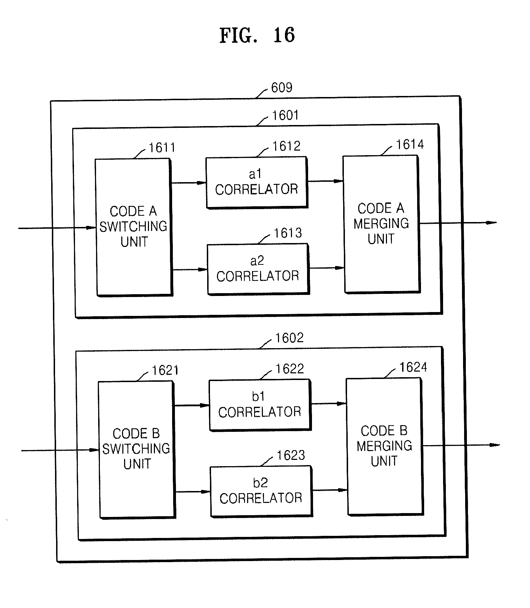

FIG. 16 is a diagram which illustrates the structure of the reception decoder 609. The reception decoder 609 of FIG. 16 includes a code A decoder 1601 and a code B decoder 1602.

The code A decoder 1601 separates code A reflected signals from among signals, and outputs the separated code A reflected signals to the image processor 610. Referring to the above description, code A reflected signals include codes which are transmitted by the transducer array 1411 in the elevation direction (i.e., the y-axis direction), and the code A reflected signals are received by the transducer array 1412 in the lateral direction (i.e., the x-axis direction). Therefore, regarding the signals separated by the code A decoder 1601, the transducer array 1411 in the elevation direction (i.e., the y-axis direction) becomes the transmitting transducer array and the transducer array 1412 in the lateral direction (i.e., the x-axis direction) becomes the receiving transducer array. Conversely, the code B decoder 1602 separates code B reflected signals from among signals, and outputs the separated code B reflected signals to the image processor 610. Regarding the code B reflected signals, the transducer array 1412 in the lateral direction (i.e., the x-axis direction) becomes the transmitting transducer array and the transducer array 1411 in the elevation direction (i.e., the y-axis direction) becomes the receiving transducer array. Hereinafter, a configuration for categorizing signals based on codes with reference to structures of the code A decoder 1601 and the code B decoder 1602 will be provided.

The code A decoder 1601 includes a code A switching unit 1611, an a1 correlator 1612, an a2 correlator 1613, and a code A merging unit 1614. Because the number of correlators may be the same as the number of codes, additional correlators, such as an a3 correlator, an a4 correlator, and so on, may be further arranged. The code A switching unit 1611 outputs signals which are output by the reception beam-forming unit 607 based on the types of code which are output by the code outputting unit 611 to the a1 correlator 1612 or to the a2 correlator 1613. In detail, the code A switching unit 1611 outputs codes which are output by the reception beam-forming unit 607 to the a1 correlator 1612 if codes which are output by the code outputting unit 611 are a1 codes, and outputs codes which are output by the reception beam-forming unit 607 to the a2 correlator 1613 if codes which are output by the code outputting unit 611 are a2 codes.

If only a1 codes are output by the code outputting unit 611, the code A switching unit 1611 may be fixed to the a1 correlator 1612. When signals which are output by the code A switching unit 1611 are received, the a1 correlator 1612 obtains signals which indicate an image of a target object which corresponds to the focal point by using the signals which are output by the code A switching unit 1611 and the a1 codes. In detail, the a1 correlator 1612 obtains signals which indicate the image of the target object which corresponds to the focal point from the signal which is output by the code A switching unit 1611 by performing a convolution calculation regarding the signals which are output by the code A switching unit 1611 and the a1 codes.

In the same regard, when signals which are output by the code A switching unit 1611 are received, the a2 correlator 1613 obtains signals which indicate an image of a target object which corresponds to the focal point by using the signals which are output by the code A switching unit 1611 and the a2 codes. In detail, the a2 correlator 1613 obtains signals which indicate the image of the target object which corresponds to the focal point from the signal which is output by the code A switching unit 1611 by performing a convolution calculation regarding the signals which are output by the code A switching unit 1611 and the a2 codes.

Equation 25 below is a general equation for performing a convolution calculation regarding x(t) and y(t). In Equation 25, t denotes time, x denotes an integral constant, and ⊗denotes convolution. x(t) denotes an electrical signal, and y(t) denotes a code signal. Furthermore, a result of the convolution is indicated by $\Psi_{xy}$, in which the subnotes $_x$ and $_y$ denote signals.

[Equation 25]

$$x(t) \otimes y(t) = x(t) * y(-t) = \int_{-\infty}^{\infty} x(t)y(t - \tau)d\tau = \Psi_{xy}$$

As described above, because the type A codes and the type B codes are orthogonal to each other, if the type A codes and the type B codes are convolved, zero may be obtained, as shown in Equation 26 below.

[Equation 26]

$$\Psi_{a1b1} = a_1(t) \otimes b_1(t) = 0$$

$$\Psi_{b1a1} = b_1(t) \otimes a_1(t) = 0$$

$$\Psi_{a2b2} = a_2(t) \otimes b_2(t) = 0$$

$$\Psi_{b2a2} = b_2(t) \otimes a_2(t) = 0$$

Further, when the same type of codes are convolved, an impulse function may be obtained, as shown in Equation 27 below.

[Equation 27]

$$\Psi_{a1a1} = a_1(t) \otimes a_1(t) = \delta(t)$$

$$\Psi_{a2a2} = a_2(t) \otimes a_2(t) = \delta(t)$$

$$\Psi_{b1b1} = b_1(t) \otimes b_1(t) = \delta(t)$$

$$\Psi_{b2b2} = b_2(t) \otimes b_2(t) = \delta(t)$$

When a signal which is output by the reception beam-forming unit 607 is indicated as x(t), x(t) includes both A type code signal components and B type code signal components. Therefore, x(t) may be expressed as shown in Equation 28 below. In Equation 28, $a_1(t)$ indicates type A codes and $b_1(t)$ indicates type B codes.
[Equation 28]

$$x(t) = \{a_1(t - t_a - t_r) + b_1(t - t_b - t_r)\}$$

The a1 correlator 1612 may obtain a signal $\Psi_{xy}$ which indicates an image of a target object which corresponds to the focal point from the signal x(t) which is output by the code A switching unit 1611 by performing a convolution calculation regarding the signal x(t) which is output by the code A switching unit 1611 and $a_1(t)$ which corresponds to the a1 codes by using Equation 29 below. In Equation 29, tr denotes a period of time for a1 codes to be transmitted by the cross-transducer arrays 1411 and 1412 and to be reflected by a target object.
[Equation 29]

$$x(t) \otimes a_1(t) = \{a_1(t - t_r) + b_1(t - t_r)\} \otimes a_1(t)$$

$$= a_1(t - t_r) \otimes a_1(t) + b_1(t - t_r) \otimes a_1(t)$$

$$= \Psi_{a1a1}$$

The b1 correlator 1622 may obtain a signal $\Psi_{xy}$ which indicates an image of a target object which corresponds to the focal point from the signal x(t) which is output by the code B switching unit 1621 by performing a convolution calculation regarding the signal x(t) which is output by the code B switching unit 1621 and $b_1(t)$ which corresponds to the b1 codes by using Equation 30 below. In Equation 30, tr denotes a period of time for b1 codes to be transmitted by the cross-transducer arrays 1411 and 1412, to be reflected by a target object, and to return.
[Equation 30]

$$x(t) \otimes b_1(t) = \{a_1(t - t_r) + b_1(t - t_r)\} \otimes b_1(t)$$
$$= a_1(t - t_r) \otimes b_1(t) + b_1(t - t_r) \otimes b_1(t)$$
$$= \Psi_{b1b1}$$

The code A merging unit 1614 outputs merged results of the a1 correlator 1612 and the a2 correlator 1613 performing the convolution to the image processor 610.

The code B decoder 1602 has the same structure as the code A decoder 1601. The code B decoder 1602 includes the code B switching unit 1621, the b1 correlator 1622, a b2 correlator 1623, and a code B merging unit 1624. Because the number of correlators may be the same as the number of codes, additional correlators, such as a b3 correlator, a b4 correlator, and so on, may be further arranged. The code B switching unit 1621 outputs a signal which has been beam-formed by the reception beam-forming unit 607 to the b1 correlator 1622 or the b2 correlator 1623. In particular, the code B switching unit 1621 outputs a code b1 signal to the b1 correlator 1622 and outputs a code b2 signai to the b2 correlator 1623. When signals are input by the code B switching unit 1621, the b1 correlator 1622 and the b2 correlator 1623 calculate a convolution integral by using a code b1 and a code b2, respectively.

The code B merging unit 1624 outputs merged results of the b1 correlator 1622 and the b2 correlator 1623 performing the convolution to the image processor 610.

A code merging unit (not shown) may be added for merging the results which are output by the code A merging unit 1614 and the code B merging unit 1624 and for outputting intensities of signals as image data to the image processor 610. In particular, the merging may simply be an average of signal intensities of the two results.

According to another exemplary embodiment, the image processor 610 may merge the results which are output by the code A merging unit 1614 and the code B merging unit 1624 and obtain brightness information which relates to 3D image pixels based on intensities of signals. A 3D image is generated based on the brightness information and is output to the image display device 103.

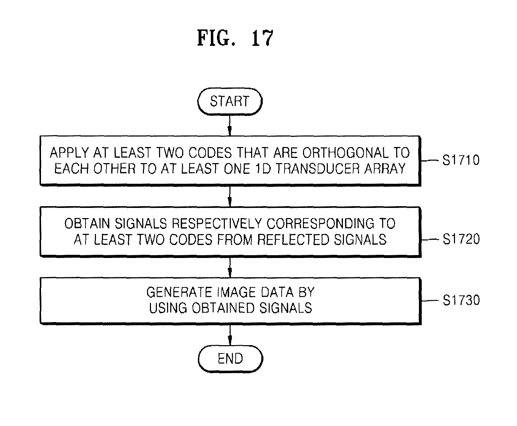

FIG. 17 is a flowchart which illustrates a method for scanning 3D ultrasound volume by using a cross-transducer array. Flereinafter, any of descriptions already given above with reference to FIGS. 8 through 16 may be omitted.

Referring to FIG. 17, in operation S1710, the transmitter 630 of FIG. 12 applies at least two codes that are orthogonal to each other to at least one 1D transducer array which is included in a 2D transducer array. The 2D transducer array may include not only the transducer arrays shown in FIGS. 5 and 7A through 7D, but also any of various other transducer arrays which include a plurality of 1D transducer arrays. Furthermore, the transmitter 630 may apply at least two orthogonal codes to 1D transducer arrays which are included in a 2D transducer array as described above with reference to FIGS. 8 through 12, or the transmitter 630 may apply one orthogonal code to an 1D transducer array which is included in a 2D transducer array as described above with reference to FIG. 14. In particular, the former corresponds to a case in which one 1D transducer array forms a plurality of transmission beam planes or performs dynamic focusing of ultrasound beams, whereas the latter corresponds to a case in which one 1D transducer array forms one transmission beam plane and performs fixed focusing of ultrasound beams. However, in the case of the latter, because codes which are orthogonal to each other are respectively applied to at least two 1D transducer arrays which are included in a 2D transducer array, resolution in the elevation direction may be improved as compared to the CA-FF technique in the related art, and 3D images may be obtained in real time, as described above.

In operation S1720, the receiver 620 of FIG. 12 obtains signals which respectively correspond to the at least two codes that are orthogonal to each other from among signals that are reflected by a target object and received by a 2D transducer array. In particular, in order to obtain signal components which relate to predetermined codes from among codes that are orthogonal to each other from among signals which are reflected by the target object, the receiver 620 performs correlation between reflected signals and the predetermined signals, In this case, due to orthogonality of the codes, signal components which relate to different codes may be removed, as described above. Furthermore, the receiver 620 performs dynamic reception focusing of the reflected signals or applies a reception delay pattern which is output by the reception signal delaying unit 606 to the reflected signals for steering a reception beam plane. The receiver 620 may perform a reception beamforming by combining signals to which the reception delay pattern is applied. Further, as described above with reference to FIGS. 8 through 16, the receiver 620 may apply at least two reception delay patterns in parallel, such that a 2D transducer array simultaneously forms a plurality of reception beam planes or such that a reception beam plane has at least two focal distances.

In operation S1730, the image processor 610 generates image data which relates to the target object by using signals which are obtained by the receiver 620 from the reflected signals. The image data which relates to the target object is generated by combining intensities of the signals which are obtained by the receiver 620 from the reflected signals. For example, the image processor 610 may categorize signals which are obtained by the receiver 620 based on focal distances, and use the averages of the signals which are categorized based on focal distances as image data which relates to the respective focal distances. For example, the image processor 610 may calculate the average of intensities of N signals which relate to a predetermined focal distance and use the average as the brightness of a B-mode ultrasound image.

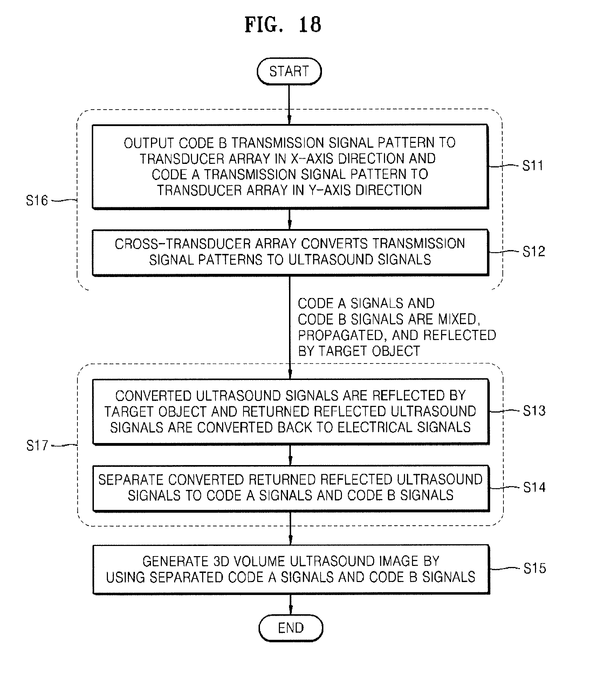

FIG. 18 is a flowchart which illustrates a method for scanning 3D ultrasound volume by using the cross-transducer array shown in FIG. 14. Referring to FIG. 18, in an operation S16, the transmitter 630 of FIG. 12 applies one of a pair of codes that are orthogonal to each other to the transducer array 1411 in the elevation direction (i.e., the y-axis direction) and applies the other one of the pair of codes to the transducer array 1412 in the lateral direction (i.e., the x-axis direction). Operation S16 includes operation S11 and operation S12.

In the operation S11, the pulser 601 generates transmission signal patterns and outputs the generated transmission signal patterns to the cross-transducer arrays 1411 and 1412. In particular, different code transmission signal patterns are output to respective linear transducer arrays of the cross-transducer array. For example, referring to FIG. 14, a code B transmission signal pattern is output to the transducer array in the x-axis direction, whereas a code A transmission signal pattern is output to the transducer array in the y-axis direction.

In the operation S12, when the transmission signal patterns are output by the pulser 601, the cross-transducer arrays 1411 and 1412 convert the transmission signal patterns to ultrasound signals and transmit the ultrasound signals to a target object. In particular, the transducer array in the x-axis direction transmits type B code ultrasound signals, whereas the transducer array in the y-axis direction transmits type A code ultrasound signals.

In operation S17, the receiver 620 obtains codes which are transmitted by the transducer array 1411 in the elevation direction (i.e., the y-axis direction) from signals that are reflected by the target object and received by the transducer array 1412 in the lateral direction (i.e., the x-axis direction) and obtains codes which are transmitted by the transducer array 1412 in the lateral direction (i.e., the x-axis direction) from that are reflected by the target object and received by the transducer array 1411 in the elevation direction (i.e., the y-axis direction). Operation S17 includes operation S13 and S14.

In S13, the cross-transducer arrays 1411 and 1412 convert the type A code ultrasound signals and the type B code ultrasound signals which are reflected by the target object back into electrical signals.

In operation S14, the reception decoder 609 separates the electrical signals into type A code signals and type B code signals.

In operation S15, the image processor 610 obtains image data from the separated type A code signals and type B code signals and generates a 3D volume ultrasound image by using the image data.

The exemplary embodiments can be written as computer programs and can be implemented in general-use digital computers that execute the programs using a transitory or non-transitory computer readable storage medium. Examples of the non-transitory computer readable storage medium include magnetic storage media (e.g., read-only memory (ROM), floppy disks, hard disks, and/or any other suitable type of magnetic storage medium), optical recording media (e.g., compact disk - ROM (CD-ROMs), or digital versatile disks (DVDs)), and any other suitable non-transitory computer readable storage medium.

In particular the invention provides the following further embodiments:

1. A method for performing three-dimensional (3D) ultrasound volume scanning by using a two-dimensional (2D) transducer array, in which a plurality of transducer elements are two-dimensionally arranged, the method comprising:

applying at least two codes that are orthogonal to each other to at least one one-dimensional (1D) transducer array which is included in the 2D transducer array, the at least one 1D transducer array having at least two transducer elements which are arranged linearly from among the plurality of transducer elements;
obtaining signals which respectively correspond to the applied at least two codes that are orthogonal to each other from signals which are reflected by a target object and received by the plurality of transducer elements; and
generating image data which relates to the target object by using the obtained signals.

2. The method of embodiment 1, wherein a number of the at least one 1D transducer array is different from a number of the at least two codes which are applied to the 1D transducer array.

3. The method of embodiment 2, wherein the applying the at least two codes comprises more than N codes that are orthogonal to one another being applied to N 1D transducer arrays which are included in the 2D transducer array.

4. The method of embodiment 1, wherein the applying the at least two codes comprises M codes that are orthogonal to one another being applied to M 1D transducer arrays which are included in the 2D transducer array.

5. The method of embodiment 1, wherein the applying the at least two codes that are orthogonal to each other comprises:

applying at least two transmission delay patterns for delaying the applied at least two codes that are orthogonal to each other to the applied at least two codes;

combining the applied at least two codes to which the at least two transmission delay patterns have been applied to one another; and

outputting a result of the combining to the at least one 1D transducer array.

6. The method of embodiment 5, wherein the applying the at least two transmission delay patterns comprises respectively applying different transmission delays to each of the applied at least two codes such that ultrasound waves from the at least one 1D transducer array are focused on at least two planes.

7. The method of embodiment 5, wherein the applying the at two transmission delay patterns comprises respectively applying different transmission delays to the applied at least two codes such that an ultrasound beam which is focused on the target object by the 1D transducer array has at least two focal distances in an axial direction.

8. The method of embodiment 1, wherein the applying the at least two codes comprises applying a first code to a first 1D transducer array which is included in the 2D transducer array, wherein transducer elements which are included in the first 1D transducer array are arranged in a first direction, and

applying a second code to a second 1D transducer array which is included in the 2D transducer array, wherein transducer elements which are included in the second 1D transducer array are arranged in a second direction.

9. The method of embodiment 8, wherein the first direction intersects with the second direction.

10. The method of embodiment 8, wherein a signal which corresponds to the applied first code is obtained from a signal which is reflected by the target object and is received by the second 1D transducer array, and

a signal which corresponds to the applied second code is obtained from a signal which is reflected by the target object and is received by the first 1D transducer array.

11. The method of embodiment 1, wherein a signal which corresponds to a predetermined code from among the applied at least two codes is obtained by calculating a correlation between the predetermined code and the signals which are reflected by the target object.

12. The method of embodiment 1, wherein the obtaining the signals comprises:

converting ultrasound signals which are respectively received by each of the plurality of transducer elements to electric signals;

applying at least one reception delay pattern for delaying the electric signals to the electric signals;

combining the electric signals to which the at least one reception delay pattern is applied; and

calculating the signals which respectively correspond to the applied at least two codes that are orthogonal to each other from the combined electric signals.

13. The method of embodiment 12, wherein the applying the at least one reception delay pattern comprises delaying the electric signals by using at least two reception delay patterns such that the signals which are reflected by the target object and received by the plurality of the transducer elements form at least two reception beam planes at the target object.

14. The method of embodiment 12, wherein the applying the at least one reception delay pattern comprises delaying the electric signals by using at least two reception delay patterns such that the signals which are reflected by the target object and received by the plurality of the transducer elements have at least two focal distances.

15. The method of embodiment 12, wherein the combining the electric signals comprises combining electric signals to which a same reception delay pattern is applied.

16. The method of embodiment 1, wherein the generating the image data comprises combining intensities of the obtained signals.

17. A three-dimensional (3D) ultrasound volume scanning apparatus comprising:

a two-dimensional (2D) transducer array, in which a plurality of transducer elements are two-dimensionally arranged;

a transmitter which is configured to apply at least two codes that are orthogonal to each other to at least one one-dimensional (1D) transducer array, the at least one 1D transducer array having at least two transducer elements which are arranged linearly from among the plurality of transducer elements;

a receiver which is configured to obtain signals which respectively correspond to each other from signals which are reflected by a target object and received by the plurality of transducer elements; and

an image processor which is configured to generate image data which relates to the target object by using the obtained signals.

18. The 3D ultrasound volume scanning apparatus of embodiment 17, wherein the transmitter is further configured to apply more than N codes that are orthogonal to one another to N 1D transducer arrays which are included in the 2D transducer array.

19. The 3D ultrasound volume scanning apparatus of embodiment 17, wherein the transmitter is further configured to apply M codes that are orthogonal to one another to M 1D transducer arrays which are included in the 2D transducer array.

20. The 3D ultrasound volume scanning apparatus of embodiment 17, wherein the transmitter comprises:

an encoder which is configured to at two transmission delay patterns for the applied at two codes to the applied at least two codes, and

a combiner which is configured to combine the applied at least two codes to which the at least two transmission delay patterns have been applied and a result of the combining to the at least one 1D transducer array.

21. The 3D ultrasound volume scanning apparatus of embodiment 20, wherein the encoder is further configured to apply different transmission delays respectively to each of the applied at least two codes such that ultrasound waves from the at least one 1D transducer array are focused on at least two planes,

22. The 3D ultrasound volume scanning apparatus of embodiment 20, wherein the encoder is further configured to apply different transmission delays respectively to each of the applied at least two codes such that an ultrasound beam which is focused on the target object by the 1D transducer array has at least two focal distances in the axial direction.

23. The 3D ultrasound volume scanning apparatus of embodiment 20, wherein the transmitter is further configured to apply a first code to a first 1D transducer array which is included in the 2D transducer array, wherein transducer elements which are included the first 1D transducer array are arranged in a first direction, and wherein the transmitter is further configured to apply a second code to a second 1D transducer array which is included in the 2D transducer array, wherein transducer elements which are included in the first 1D transducer array are arranged in a second direction.

24. The 3D ultrasound volume scanning apparatus of embodiment 23, wherein the first direction intersects with the second direction.

25. The 3D ultrasound volume scanning apparatus of embodiment 23, wherein the receiver is further configured to obtain a signal which corresponds to the applied first code from a signal which is reflected by the target object and is received by the second 1D transducer array, and wherein the receiver is further confgured to obtain a signal which corresponds to the applied second code from a signal which is reflected by the target object and is received by the first 1D transducer array.

26. The 3D ultrasound volume scanning apparatus of embodiment 17, wherein the receiver is further configured to obtain a signal which corresponds to a predetermined code from among the applied at least two codes by calculating a correlation between the predetermined code and the signals which are reflected by the target object.

27. The 3D ultrasound volume scanning apparatus of embodiment 17, wherein the receiver comprises:

a reception beam former which is configured to apply at least one reception delay pattern to electric signals which correspond to ultrasound signals which are respectively received by each of the plurality of transducer elements and to combine the electric signals to which the at least one reception delay pattern is applied; and

a decoder which is configured to calculate signals which respectively correspond to the applied at least two codes from the combined electric signals.

28. The 3D ultrasound volume scanning apparatus of embodiment 27, wherein the reception beam former is further configured to delay the electric signals by using at least two reception delay patterns such that the signals which are reflected by the target object and received by the plurality of transducer elements form at least two reception beam planes with respect to the target object.

29. The 3D ultrasound volume scanning apparatus of embodiment 27, wherein the reception beam former is further configured to delay the electric signals by using at least two reception delay patterns such that the signals which are reflected by the target object and received by the plurality of transducer elements have at least two focal distances.

30. The 3D ultrasound volume scanning apparatus of embodiment 27, wherein the reception beam former is configured to combine electric signals to which a same reception delay pattern is applied.

31. The 3D ultrasound volume scanning apparatus of embodiment 17, wherein the image processor is further configured to generate the image data by combining intensities of the obtained signals.

32. A non-transitory computer readable storage medium having stored thereon a computer program, which when executed by a computer, implements the method of any of embodiment 1.

It should be understood that the exemplary embodiments described herein should be considered in a descriptive sense only and not for purposes of limitation. Descriptions of features or aspects within each exemplary embodiment should typically be considered as available for other similar features or aspects in other exemplary embodiments.

**Claims**

1. A method for performing three-dimensional (3D) ultrasound volume scanning by using a two-dimensional (2D) transducer array, in which a plurality of transducer elements are two-dimensionally arranged, the method comprising:

applying at least two codes that are orthogonal to each other to at least one one-dimensional (1D) transducer array which is included in the 2D transducer array, the at least one 1D transducer array having at least two transducer elements which are arranged linearly from among the plurality of transducer elements; obtaining signals which respectively correspond to the applied at least two codes that are orthogonal to each other from signals which are reflected by a target object and received by the plurality of transducer elements; and generating image data which relates to the target object by using the obtained signals.

2. The method of claim 1, wherein a number of the at least one 1D transducer array is different from a number of the at least two codes which are applied to the 1D transducer array.

3. The method of claim 2, wherein the applying the at least two codes comprises more than N codes that are orthogonal to one another being applied to N 1D transducer arrays which are included in the 2D transducer array.

4. The method of claim 1, wherein the applying the at least two codes comprises M codes that are orthogonal to one another being applied to M 1D transducer arrays which are included in the 2D transducer array.

5. The method of claim 1, wherein the applying the at least two codes that are orthogonal to each other comprises:

applying at least two transmission delay patterns for delaying the applied at least two codes that are orthogonal to each other to the applied at least two codes; combining the applied at least two codes to which the at least two transmission delay patterns have been applied to one another; and outputting a result of the combining to the at least one 1D transducer array.

6. The method of claim 5, wherein the applying the at least two transmission delay patterns comprises respectively applying different transmission delays to each of the applied at least two codes such that ultrasound waves from the at one 1D transducer array are focused on at least two planes.

7. The method of claim 5, wherein the applying the at least two transmission delay patterns comprises respectively applying different transmission delays to the applied at least two codes such that an ultrasound beam which is focused on the target object by the 1D transducer array has at least two focal distances in an axial direction.

8. The method of claim 1, wherein the applying the at least two codes comprises applying a first code to a first 1D transducer array which is included in the 2D transducer array, wherein transducer elements which are included in the first 1D transducer array are arranged in a first direction, and applying a second code to a second 1D transducer array which is included in the 2D transducer array, wherein transducer elements which are included in the second 1D transducer array are arranged in a second direction.

9. The method of claim 8, wherein the first direction intersects with the second direction.

10. The method of claim 8, wherein a signal which corresponds to the applied first code is obtained from a signal which is reflected by the target object and is received by the second 1D transducer array, and a signal which corresponds to the applied second code is obtained from a signal which is reflected by the target object and is received by the first 1D transducer array.

11. The method of claim 1, wherein a signal which corresponds to a predetermined code from among the applied at least two codes is obtained by calculating a correlation between the predetermined code and the signals which are reflected by the target object.

12. The method of claim 1, wherein the obtaining the signals comprises:

converting ultrasound signals which are respectively received by each of the plurality of transducer elements to electric signals;
applying at least one reception delay pattern for delaying the electric signals to the electric signals;
combining the electric signals to which the at least one reception delay pattern is applied; and
calculating the signals which respectively correspond to the applied at least two codes that are orthogonal to each other from the combined electric signals.

13. The method of claim 12, wherein the applying the at least one reception delay pattern comprises delaying the electric signals by using at least two reception delay patterns such that the signals which are reflected by the target object and received by the plurality of the transducer elements form at least two reception beam planes at the target object.

14. The method of claim 12, wherein the applying the at least one reception delay pattern comprises delaying the electric signals by using at least two reception delay patterns such that the signals which are reflected by the target object and received by the plurality of the transducer elements have at least two focal distances.

15. The method of claim 12, wherein the combining the electric signals comprises combining electric signals to which a same reception delay pattern is applied.

16. The method of claim 1, wherein the generating the image data comprises combining intensities of the obtained signals.

17. A three-dimensional (3D) ultrasound volume scanning apparatus comprising:

a two-dimensional (2D) transducer array, in which a plurality of transducer elements are two-dimensionally arranged;
a transmitter which is configured to apply at least two codes that are orthogonal to each other to at least one one-dimensional (1D) transducer array, the at least one 1D transducer array having at least two transducer elements which are arranged linearly from among the plurality of transducer elements;
a receiver which is configured to obtain signals which respectively correspond to each other from signals which are reflected by a target object and received by the plurality of transducer elements; and
an image processor which is configured to generate image data which relates to the target object by using the obtained signals.

18. The 3D ultrasound volume scanning apparatus of claim 17, wherein the transmitter is further configured to apply more than N codes that are orthogonal to one another to N 1D transducer arrays which are included in the 2D transducer array.

19. The 3D ultrasound volume scanning apparatus of claim 17, wherein the transmitter is further configured to apply M codes that are orthogonal to one another to M 1D transducer arrays which are included in the 2D transducer array.

20. The 3D ultrasound volume scanning apparatus of claim 17, wherein the transmitter comprises:

an encoder which is configured to apply at least two transmission delay patterns for delaying the applied at least two codes to the applied at least two codes; and
a combiner which is configured to combine the applied at least two codes to which the at least two transmission delay patterns have been applied and a result of the combining to the at least one 1D transducer array.

21. The 3D ultrasound volume scanning apparatus of claim 20, wherein the encoder is further configured to apply different transmission delays respectively to each of the applied at least two codes such that ultrasound waves from the at least one 1D transducer array are focused on at least two planes.

22. The 3D ultrasound volume scanning apparatus of claim 20, wherein the encoder is further configured to apply different transmission delays respectively to each of the applied at least two codes such that an ultrasound beam which is focused on the target object by the 1D transducer array has at least two focal distances in the axial direction.

23. The 3D ultrasound volume scanning apparatus of claim 20, wherein the transmitter is further configured to apply a first code to a first 1D transducer array which is included in the 2D transducer array, wherein transducer elements which are included the first 1D transducer array are arranged in a first direction, and wherein the transmitter is further configured to apply a second code to a second 1D transducer array which is included in the 2D transducer array, wherein transducer elements which are included in the first 1D transducer array are arranged in a second direction.

24. The 3D ultrasound volume scanning apparatus of claim 23, wherein the first direction intersects with the second direction.

25. The 3D ultrasound volume scanning apparatus of claim 23, wherein the receiver is further configured to obtain a signal which corresponds to the applied first code from a signal which is reflected by the target object and is received by the second 1D transducer array, and wherein the receiver is further configured to obtain a signal which corresponds to the applied second code from a signal which is reflected by the target object and is received by the first 1D transducer array.

26. The 3D ultrasound volume scanning apparatus of claim 17, wherein the receiver is further configured to obtain a signal which corresponds to a predetermined code from among the applied at least two codes by calculating a correlation between the predetermined code and the signals which are reflected by the target object.

27. The 3D ultrasound volume scanning apparatus of claim 17, wherein the receiver comprises:

   a reception beam former which is configured to apply at least one reception delay pattern to electric signals which correspond to ultrasound signals which are respectively received by each of the plurality of transducer elements and to combine the electric signals to which the at least one reception delay pattern is applied; and
   a decoder which is configured to calculate signals which respectively correspond to the applied at least two codes from the combined electric signals.

28. The 3D ultrasound volume scanning apparatus of claim 27, wherein the reception beam former is further configured to delay the electric signals by using at least two reception delay patterns such that the signals which are reflected by the target object and received by the plurality of transducer elements form at least two reception beam planes with respect to the target object.

29. The 3D ultrasound volume scanning apparatus of claim 27, wherein the reception beam former is further configured to delay the electric signals by using at least two reception delay patterns such that the signals which are reflected by the target object and received by the plurality of transducer elements have at least two focal distances.

30. The 3D ultrasound volume scanning apparatus of claim 27, wherein the reception beam former is configured to combine electric signals to which a same reception delay pattern is applied.

31. The 3D ultrasound volume scanning apparatus of claim 17, wherein the image processor is further configured to generate the image data by combining intensities of the obtained signals.

32. A non-transitory computer readable storage medium having stored thereon a computer which when executed by a computer, implements the of any of claim 1.

FIG. 1

FIG. 2

FIG. 3

Y-AXIS
(ELEVATION DIRECTION)

2021,2022

21

φ

203

204

Z-AXIS
(AXIAL DIRECTION)

X-AXIS
(LATERAL DIRECTION)

FIG. 4

Y-AXIS
(ELEVATION DIRECTION)

Z
(LATERAL DIRECTION)

RF1      RF1      RF3

FIG. 5

FIG. 6

FIG. 7A

FIG. 7B

FIG. 7C

FIG. 7D

FIG. 8

FIG. 9

## FIG. 10

## FIG. 11

FIG. 12

FIG. 13A

EP 2 674 777 A1

FIG. 13B

TIME
(25ns Samples)

EP 2 674 777 A1

FIG. 13C

FIG. 13D

TRANSMITTED ULTRASOUND WAVE
PULSER OUTPUT

FIG. 14

1411

1412

TYPE A1 CODE TRANSMISSION

TYPE B1 CODE TRANSMISSION

FIG. 15

1411

1412

TYPE A1 CODE RECEPTION

TYPE B1 CODE RECEPTION

FIG. 16

FIG. 17

```
                    ( START )
                        |
                        v
+----------------------------------------------+
| APPLY AT LEAST TWO CODES THAT ARE ORTHOGONAL TO |--- S1710
| EACH OTHER TO AT LEAST ONE 1D TRANSDUCER ARRAY  |
+----------------------------------------------+
                        |
                        v
+----------------------------------------------+
| OBTAIN SIGNALS RESPECTIVELY CORRESPONDING TO |
| AT LEAST TWO CODES FROM REFLECTED SIGNALS    |--- S1720
+----------------------------------------------+
                        |
                        v
+----------------------------------------------+
|         GENERATE IMAGE DATA BY               |
|         USING OBTAINED SIGNALS               |--- S1730
+----------------------------------------------+
                        |
                        v
                    ( END )
```

# FIG. 18

START

S16

OUTPUT CODE B TRANSMISSION SIGNAL PATTERN TO
TRANSDUCER ARRAY IN X-AXIS DIRECTION AND
CODE A TRANSMISSION SIGNAL PATTERN TO
TRANSDUCER ARRAY IN Y-AXIS DIRECTION — S11

CROSS-TRANSDUCER ARRAY CONVERTS TRANSMISSION
SIGNAL PATTERNS TO ULTRASOUND SIGNALS — S12

CODE A SIGNALS AND
CODE B SIGNALS ARE MIXED,
PROPAGATED, AND REFLECTED
BY TARGET OBJECT

S17

CONVERTED ULTRASOUND SIGNALS ARE REFLECTED BY
TARGET OBJECT AND RETURNED REFLECTED ULTRASOUND
SIGNALS ARE CONVERTED BACK TO ELECTRICAL SIGNALS — S13

SEPARATE CONVERTED RETURNED REFLECTED ULTRASOUND
SIGNALS TO CODE A SIGNALS AND CODE B SIGNALS — S14

GENERATE 3D VOLUME ULTRASOUND IMAGE BY
USING SEPARATED CODE A SIGNALS AND CODE B SIGNALS — S15

END

Europäisches
Patentamt

European
Patent Office

Office européen
des brevets

**EUROPEAN SEARCH REPORT**

Application Number

EP 13 17 1576

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/111846 A1 (SCHMIED M; STEINBACHER F) 26 May 2005 (2005-05-26) <br><br> * abstract; figures 4, 6 * <br> * paragraphs [0016], [0017], [0019] * <br> * paragraphs [0021], [0022] * <br> * paragraph [0032] - paragraph [0041] * <br> ----- | 1,4-7, 11-17, 19-22, 26-32 | INV. <br> G01S7/52 <br> G01S15/89 |
| X | US 2003/018261 A1 (BAE MOO HO [KR]) 23 January 2003 (2003-01-23) | 1-3, 8-18, 23-32 | |
| Y | * abstract; figures 2A, 2B * <br> * paragraph [0040] - paragraph [0043] * <br> * paragraph [0054] - paragraph [0063] * <br> * paragraph [0074] - paragraph [0075] * <br> ----- | 8-10, 23-25 | |
| X | US 5 598 206 A (BULLIS JAMES K [US]) 28 January 1997 (1997-01-28) | 1-3,5-7, 11-18, 20-22, 26-32 | |
| Y | * abstract; figures 2, 4 - 6 * <br> * column 1, line 5 - line 8 * <br> * column 7, line 55 - column 8, line 8 * <br> * column 9, line 54 - column 10, line 32 * <br> * column 11, line 50 - column 12, line 64 * <br> * column 13, line 26 - line 27 * <br> ----- | 8-10, 23-25 | TECHNICAL FIELDS SEARCHED (IPC) <br><br> G01S |

The present search report has been drawn up for all claims

| Place of search | Date of completion of the search | Examiner |
|---|---|---|
| Munich | 10 September 2013 | Knoll, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 13 17 1576

This annex lists the patent family members relating to the patent documents cited in the above-mentioned European search report.
The members are as contained in the European Patent Office EDP file on
The European Patent Office is in no way liable for these particulars which are merely given for the purpose of information.

10-09-2013

| Patent document cited in search report | | Publication date | Patent family member(s) | | Publication date |
|---|---|---|---|---|---|
| US 2005111846 | A1 | 26-05-2005 | CN | 1636518 A | 13-07-2005 |
| | | | FR | 2862763 A1 | 27-05-2005 |
| | | | JP | 2005152628 A | 16-06-2005 |
| | | | US | 2005111846 A1 | 26-05-2005 |
| US 2003018261 | A1 | 23-01-2003 | JP | 2003010181 A | 14-01-2003 |
| | | | KR | 20020083011 A | 01-11-2002 |
| | | | US | 2003018261 A1 | 23-01-2003 |
| US 5598206 | A | 28-01-1997 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5305756 A
- US 5417219 A
- US 5901708 A